# EUROPEAN PATENT SPECIFICATION

(11) **EP 1 088 069 B1**
(45) Date of publication and mention of the grant of the patent: **11.05.2005**
(21) Application number: 99927981.3
(22) Date of filing: 11.06.1999
(51) Int. Cl.: C12N 15/12, C12N 5/10, C12N 1/21, C07K 14/71, C07K 16/28, G01N 33/50, A61K 38/17

(54) **ANGIOSTATIN-BINDING PROTEIN**
ANGIOSTATIN-BINDENDES PROTEIN
PROTEINE SE LIANT A L'ANGIOSTATINE

(30) Priority: 15.06.1998 SE 9802130; 15.06.1998 US 89266 P; 17.12.1998 SE 9804372; 29.12.1998 US 114386 P
(43) Date of publication of application: 04.04.2001
(73) Proprietor: BioInvent International AB, 223 70 LUND (SE)
(72) Inventor: HOLMGREN, Lars, S-167 53 Bromma (SE); TROYANOVSKY, Boris, 163 74 Spanga (SE)
(74) Representative: Lettström, Richard Wilhelm
(86) International application number: PCT/EP1999/004109
(87) International publication number: WO 1999/066038

(56) References cited:
- EP-A- 0 206 400
- KOST C ET AL: "Limited plasmin proteolysis of vitronectin. Characterization of the adhesion protein as morpho-regulatory and angiostatin-binding factor" EUR J BIOCHEM, vol. 236, no. 2, 1 March 1996 (1996-03-01), pages 682-688, XP002121364
- DATABASE EMSTS E.M.B.L. Databases Accession Number: G16015, 25 January 1996 (1996-01-25) MURRAY M ET AL: "Human STS CHLC.GCT14C05.P16699" XP002121366
- CLAESSON-WELSH L ET AL: "Angiostatin induces endothelial cell apoptosis and activation of focal adhesion kinase independently of the integrin-binding motif RGD" PROCEEDINGS OF THE NATIONAL ACADEMY OF SCIENCES OF USA., vol. 95, no. 10, 12 May 1998 (1998-05-12), pages 5579-5583, XP002121365
- MOSER T EL AL: "Angiostatin binds ATP synthase on the surface of human endothelial cells" PROCEEDINGS OF THE NATIONAL ACADEMY OF SCIENCES OF USA., vol. 96, no. 6, 16 March 1999 (1999-03-16), pages 2811-2816, XP002121371

## Description

### Technical field

The present invention relates to the field of angiogenesis, and more specifically to novel molecules, such as proteins and peptides, whereby novel anti-angiogenic substances may be developed. The invention also relates to methods for developing such substances.

### Background

Almost all of the tissue of a mammalian body comprises a fine mesh of very thin blood vessels, each of which is thinner than a human hair. Usually, neither the number nor the size of these vessels increase, since the division of the endothelial cells covering the vessels is slow, actually up to several years. The exceptions are for example during wound healing and menstruation, when the vessels grow rapidly. However, that is during a limited period of time and the cell division ceases thereafter.

The generation of new blood vessels from existing ones is called angiogenesis. Angiogenesis has been associated to cancer and the formation of tumors as well as to other conditions, such as diabetes retinopathy, rheumatoid arthritis and even some inflammatory conditions. Accordingly, a considerable research effort is made world-wide to find ways of preventing and inhibiting the angiogenic process. If this were possible, tumor growth could be controlled and useful therapies could be developed regarding the above mentioned conditions.

There exists numerous pieces of evidence showing that tumors are depending on *de novo* formation of blood vessels for expansion beyond a mass of a few mm³. The angiogenesis is triggered by factors secreted by the tumor cells. It has recently been discovered that tumors through unleashed proteolytic activity generates peptide fragments, which show anti-angiogenic activities. One example is the molecule angiostatin, which is a fragment of plasminogen.

Plasminogen is a substance in blood plasma which, when activated, forms plasmin or fibrinolysin, an enzyme involved in the coagulation of blood. Plasminogen itself lacks any detectable anti-angiogenic activity. It has been found (Judah Folkman ct al, Harvard Medical School, Boston) that a part of this endogenous protein, more specifically the first four kringle domains, is capable of preventing the endothelial cells from dividing. This part of plasminogen has been denoted angiostatin, and a great deal of research within this field is centred around this molecule. The prior art has shows that angiostatin inhibits endothelial cells specifically *in vitro* and blocks angiogenesis *in vivo*. Systemic treatments with subcutaneous injections of angiostatin induces *in vivo* dormancy in a wide range of tumors in SCID mice (O'Reilly et al., Nature Med., vol. 2, p. 689, 1996). No detectable toxicity has been detected in these animals even after months of treatment. Angiostatin shows two levels of specificity: it induces apoptosis specifically in endothelial cells *in vitro* (Claesson-Welsh et al., Proc. Natl. Acad. Sci., USA. vol. 95, p. 5579, 1998) and only affects endothelial cells active in angiogenesis *in vivo*. It has not shown to negatively affect cells in established vessels.

Angiostatin does indeed exhibit some advantageous properties, *inter alia* as it is an endogenous substance. However, the disadvantages associated with its possible use for medical purposes cannot be neglected. One is that the half life thereof is very short, it may be counted in hours, thereby requiring a frequent administration thereof. This far, the efficiency thereof has proven to be rather low, which fact necessitates the use of large doses thereof. These two disadvantages are in themselves strong motives for directing further research towards the finding of alternative, smaller and/or more efficient molecules to be used as medicaments.

### Summary of the invention

The present invention solves the problems associated with angiostatin as defined above by providing a human protein, which has been named "ABP-1", defined by its ability to bind a fragment of plasminogen, preferably the first four Kringle domains (K1-K4) thereof, the said fragment being characterized by anti-angiogenic biological activity.

ABP-1 comprises an amino acid sequence having a sequence homology greater than at least 80% to SEQ ID NO. 2.

Also encompassed in the present invention are the homologs of ABP-1 in other species, especially in other mammals, having a sequence homology greater than at least 80% to SEQ ID NOs. 2, 3 or 4.

In a further aspect, the invention provides isolated nucleic acid molecules comprising a sequence that codes for ABP-1 or for a polypeptide substantially similar to ABP-1, i.e. having a sequence homology greater than at least 80% to SEQ ID NOs. 2, 3 or 4.

The present invention also includes screening methods for identifying a compound capable of interacting with ABP-1 or its variants and fragments. The screening method can be in any configuration well known to those skilled in the art.

It is still a further object of the present invention to provide antibodies directed against epitopes present in ABP-1 or its variants and fragments as well as cell producing the antibody.

It is a further object of the present invention to provide a vector comprising the nucleotide sequence of ABP-1 or its variants and fragments.

It is a further object of the present invention to provide a cell containing the above vector.

### Definitions

In the present application, the following terms are used in the meanings defined below.

As used herein, the term "angiogenesis" relates to the generation of new blood vessels into a tissue or organ. As mentioned above, under normal physiological conditions, humans and animals undergo angiogenesis only in very specific restricted situations. For example, angiogenesis is normally observed in wound healing, fetal and embryonal development and formation of the corpus luteum and placenta.

The term "endothelium" relates to the thin layer of flat epithelial cells, that lines serous cavities, lymph vessels and blood vessels. The term "endothelial inhibiting activity" relates to the capability of inhibiting the growth of endothelial capillary endothelial cells.

The term "an angiogenesis associated protein" relates to a protein capable of interacting in the angiogenesis, such as a receptor binding an anti-angiogenic substance.

The term "substantially similar", when used in reference to the amino acid sequence of SEQ ID NO.2, SEQ ID NO.3 and SEQ ID NO.4, means an amino acid sequence having an high degree of sequence homology to SEQ ID NO.2, SEQ ID NO.3 and SEQ ID NO.4. A high degree of homology means at least approximately 80% amino acid homology, preferably at least approximately 90% amino acid homology, more preferably at least approximately 95% amino acid homology and most preferably at least approximately 98% amino acid homology.

The term "specifically hybridizing to" refers to the binding, duplexing or hybridizing of a molecule only to a particular nucleotide sequence under stringent conditions when the sequence is present in a complex mixture (e.g. total cellular) of DNA or RNA. The term "stringent conditions" relates to conditions under which a probe will hybridize to its target subsequence, but to no other sequences. Stringent conditions are sequence-dependent and will be different in different circumstances. Longer sequences hybridize specifically at higher temperatures. Generally, stringent conditions are selected to be about 5°C lower than the thermal melting point (Tm) for the specific sequence at a defined ionic strength and pH. The Tm is the temperature (under defined ionic strength, pH and nucleic acid concentration) at which 50% of the probes complementary to the target sequence hybridize to the target sequence at equilibrium. (As the target sequences are generally present in excess, at Tm, 50% of the probes are occupied at equilibrium). Typically, stringent conditions will be those in which the salt concentration is less than about 1.0 M Na ion, typically about 0.01 to 1.0 M Na ion (or other salts) at pH 7.0 to 8.3 and the temperature is at least about 30°C for short probes (e.g. 10 to 50 nucleotides) and at least about 60°C for longer probes. Stringent conditions may also be achieved with the addition of destabilizing agents, such as formamide.

### Brief description of the drawings

Figure 1 shows the binding of ¹²⁵I-labelled angiostatin and plasminogen to bovine microcapillary endothelial cells *in vitro*. Units in abscissa represent the fold molar excess of cold angiostatin and plasminogen, respectively.
Figure 2 shows the strategy for isolating angiostatin binding proteins.
Figure 3 shows the growth of positive yeast clones under selective conditions.
Figure 4 compares the binding of recombinant "Big-3" to angiostatin and plasminogen, respectively.
Figure 5 is a map over "Big-3".
Figure 6 shows the open reading frame (Frame 2) of the gene encoding the angiostatin receptor according to the invention. The other possible frames do not produce any putative protein.
Figure 7 shows the expression patterns of fetal and adult mRNA as well as endothelial cells.
Figure 8 shows the relative quantitation of ABP-1 gene expression in different tissue. See experimental section for more details.
Figure 9 illustrates (A) the cellular localization of GFP-tagged ABP-1 receptor in transiently transfected HeLa cells and (B) the reorganization of GFP-labeled ABP-1 after incubation with angiostatin. Figure 9C shows the binding of angiostatin to ABP-1. See the experimental section for more details. Figure 9D is an immunostaining of ABP-1 in Human umbilical cord endothelial (HUVE) cells together with staining against F-actin with rhodamin-labelled phalloidin. ABP-1 is localized in focal adhesions and membrane ruffles (arrows).
Figure 10 illustrates the down regulation of ABP-1 associated kinase activity after addition of angiostatin.
Figure 11 is an autoradiography of an SDS-PAGE demonstrating that ABP-1 mediates angiostatin-induced focal adhesion kinase activity. See experimental section for more details.

### Detailed description of the invention

The present invention relates to an isolated human angiogenesis-associated protein capable of binding a fragment of plasminogen, preferably an N-terminal fragment, such as kringle domains 1 to 4 and/or kringle 5. Thus, the protein of the invention acts as a receptor of plasminogen fragments, in particular as a receptor of angiostatin and/or kringle 5 domain of plasminogen. The protein of the invention can be synthesized by biological or chemical methods (e.g. recombinant gene expression and peptide synthesis). Recombinant techniques include gene cloning or amplification by *in vitro* methods, such as the polymerase chain reaction (PCR), the ligase chain reaction (LCR), the transcription-based amplification system (TAS) or the self-sustained sequence replication system (SSR). A wide variety of cloning and *in vitro* amplification methodologies are well-known to those skilled in the art. Examples of these techniques are *e*.*g*. found in Berger and Kimmel. *Guide to Molecular Cloning Techniques*, Methods in Enzymology 152 Academic Press. Inc., San Diego. CA (Berger). The present invention includes proteins which comprise amino acid sequences substantially similar to those shown in SEQ ID NO.2, SEQ ID NO.3 and SEQ ID NO.4. A comparison of the protein sequence of SEQ ID NO. 2 with the sequences present in all available data bases showed a homology of 23.7% with a hypotetical protein of *Caenorhabditis elegans* encoded by a putative open reading frame located in the central cluster of chromosome III of this organism (described in R. Wilson et al., Nature, vol.368. 3 March 1994. p. 32-38). No function has been attributed to this hypotetical protein.

According to the definition given above, it is to be understood that all polypeptides capable of binding a fragment of plasminogen and having an amino acid sequence which has at least approximately 80% sequence homology, preferably approximately 90% sequence homology. more preferably approximately 95% sequence homology and most preferably approximately 98% sequence homology to SEQ ID Nos.2, 3 or 4, are contemplated as being included in the present invention. These variant forms may result, e.g., from alternative splicing or differential expression in different tissue of the same source organism. The variant forms may be characterized by, e.g., amino acid insertion, deletion or substitution. A preferred variant form of ABP-1 is illustrated in SEQ ID NO. 3 (see below).

A particularly preferred embodiment of the present invention is a fragment of ABP-1 named Big-3 which will be described in greater details below and which includes the angiostatin-hinding domain of ABP-1 (SEQ ID NO. 4)

In a further embodiment, the present invention provides peptides which comprises at least about 5, preferably at least about 10 contiguous amino acid residues of SEQ ID NO 2 or any variant or fragment thereof. However, the most advantageous size of the peptide will be depending on the intended future use thereof and the present invention is not limited to the above stated number of amino acids residues.

Also included in the present invention are homologs of ABP-1 and Big-3 in other species, in particular in other mammals. The term "homolog" refers to proteins exerting substantially the same biological function of the proteins of the invention, regardless of the homology existing between the corresponding amino acid sequences.

In another aspect, the present invention relates to isolated nucleic acid molecules comprising a sequence that codes for the proteins and peptides of the invention, including the variants, fragments and homologs as defined above. In a preferred embodiment, the nucleic acid molecule has the sequence of SEQ ID NO. 1. This sequence presents a 5' (from nucleotide 1 to nucleotide 796) and a 3' (from nucleotide 2825 to nucleotide 6463) untranslated regions. In another preferred embodiment the nucleic acid molecule has the sequence from nucleotide 797 to nucleotide 2824 of SEQ ID NO. 1. In another preferred embodiment the nucleic acid sequence encodes the ABP-1 fragment named Big-3 (illustrated in SEQ ID NO. 4) and it comprises the nucleotide sequence from position 2180 to position 2608 of SEQ ID NO. 1. All the nucleotide sequences encoding the polypeptides of the invention and differing from the nucleotide sequence of SEQ ID NO. 1 or fragment thereof by way of the degeneracy of the genetic code, are considered part of the invention. Sequencing analysis has revealed the presence of a possible polymorfism in the codon 1199-1201 of SEQ ID NO. 1, wherein a codon for Asn, Ser or Asp may be present: a further region corresponding to nucleotide positions 1238 to 1246 of SEQ ID NO. 1 has been found to code for the tripeptide Glu-Leu-Ala or for the tripeptide Thr-Trp-Pro. These variations in the amino acid sequence of ABP-1 are illustrated in SEQ ID NO. 3 which constitutes another preferred protein of the invention. Also included in the present invention are nucleotide molecules encoding a homolog of ABP-1 or fragment thereof.

The present invention further comprises a nucleic acid capable of specifically hybridizing, under stringent conditions, to any one of the nucleic acid molecules of the invention described above.

Where the nucleic acids according to the invention are to be used as probes, it is often desirable to label the sequences with detectable markers. Such markers may include any composition detectable by spectroscopic, photochemical, biochemical, immunochemical, electrical, optical or chemical means. The markers may be incorporated by any of a number of means well known to those of skill in the art. Methods for detecting such markers are also well known in the art and disclosed in the literature. The probes find a useful application in diagnostic application, for example to detect and measure ABP-1 biosynthesis in tissues and cells.

In another aspect, the present invention provide screening methods or assays, wherein molecules that exhibit the same properties as angiostatin and/or kringle 5 are screened for. In a typical screening method a compound capable of activating the angiostatin signal transduction pathway is identified through a high throughput cell-based screen. Such screen rely on a reporter gene driven by an angiostatin responsive element that is stably transfected into an angiostatin-responsive cell line. The responsive element is linked to a reporter gene, e.g. the gene for luciferase; upon binding of the compound to the protein of the invention, the intracellular pathway is activated thus causing an increase in the reporter gene activity that can be detected.

The compounds identified through the screening method hereby described are within the scope of the present invention. Such compounds are preferably low molecular weight molecules of peptidic or non-peptidic nature. Thanks to their small size they are more practical for use for medicinal purposes, compared to angiostatin. These molecules and the uses thereof are described in more detail below.

The proteins and peptides according to the invention may be synthesized using standard chemical peptide synthesis techniques. For solid phase synthesis, see *e*.*g*. Barany and Merrifield, *Solid-Phase Peptide Synthesis*, pp 3-284 in *The Peptides*: Analysis, Synthesis Biology, Vol. 2: *Special Methods in Peptide Synthesis*, Part A.

Preferably, the proteins, peptides and polypeptides according to the invention are synthesized using recombinant DNA methodology, which generally involves creating a DNA sequence that encodes the protein or peptide, placing the DNA in an expression cassette under the control of a particular promoter, expressing the protein or peptide in a host, isolating the expressed protein or peptide and, if required, renaturing the product. Once expressed, the recombinant peptides or proteins can be purified according to standard procedures in the art. Thus, another aspect of the present invention is a vector, such as a virus or a plasmid, comprising a nucleic acid according to the invention. Further, the invention also encompasses a recombinant cell transformed or transfected with the said vector expressing the present protein or peptide as well as a recombinant cell expressing the present antibody, which will be defined in more detail below. Vectors encoding the peptides and proteins according to the invention are useful in expressing those molecules to provide immunogens for antibody production. The vectors according to the invention are also useful for transforming cells *in vitro* or *in vivo* to express the present peptides and proteins. Cells expressing any one of the present nucleic acids, such as the gene defined by SEQ ID NO 1. may be used in a wide variety of contexts and are also within the scope of the present invention. Such cells may be eucaryotic or procaryotic.

The proteins and peptides according to the invention can be used as antigens for raising antibodies against the same. Consequently, the invention also encompasses an antibody which specifically binds a peptide according to the invention. Such antibodies are useful for immunoassays, e.g. for the isolation of peptides or polypeptides. The peptides according to the invention may also be used in assays, such as amplification specific assays, immunological assays *etc*.

The antibodies according to the invention may be monoclonal or polyclonal and include individual, allelic, strain or species variants, or fragments thereof, both in their naturally occurring (full-length) forms and recombinant forms. Additionally, the antibodies are raised to the present peptides or polypeptides in either their native configuration or in non-native configurations. Anti-idiotypic antibodies can also be generated. Many methods of making antibodies are known to persons skilled in the art. For techniques for preparing monoclonal antibodies, see *e*.*g*. Stiites et al (eds.). *Basic and Clinical Immunology* (4^{th} ed). Lange Medical Publications, Los Altos, CA, and references cited therein. For techniques that involve selection of libraries of recombinant antibodies in phage or similar vectors, see *e*.*g*. Huse *et al*. (1989) *Science* 246:1275-1281.

The molecules according to the invention may be used in pharmaceutical preparations, especially for the treatment and/or prevention of angiogenesis related disorders. Accordingly, the invention relates to a peptide, polypeptide, protein or antibody according to the invention for use as a medicament as well as to the use of said molecules in the manufacture of a medicament directed towards an angiogenesis related disease or disorder. The invention also relates to a pharmacological preparation comprising a molecule according to the invention together with a pharmaceutically acceptable carrier. The molecules used as medicaments according to the invention may be anyone of the above described peptides, polypeptides, proteins or antibodies as well as any novel substance identified in a screening method using the same and described above.

Accordingly, the most preferred use of the present molecules is in assays, wherein novel substances are screened for. Compounds may be identified which exhibit similar anti-angiogenic effects as angiostatin and/or kringle 5, but which are smaller, more efficient and preferably exhibits a longer half time in a human or animal body than angiostatin. The shorter half time may be due to a lower tendency to be degraded by proteases. When an organic compound is designed, a molecule according to the invention is used as a "lead" compound. The design of mimetics to known pharmaceutically active compounds is a well known approach in the development of pharmaceuticals based on such "lead" compounds. Mimetic design, synthesis and testing are generally used to avoid randomly screening a large number of molecules for a target property.

Thus, such novel molecules are preferably used as medicaments that may be administered in much lower doses than angiostatin, and which may be administered less frequently, such as *e*.*g*. once every fourteen days, which is to be compared to the half time of angiostatin which is about ten times shorter. In a particular embodiment, the novel molecules identified by the screening methods according to the invention are low molecular weight organic molecules. in which case a pharmaceutical preparation may be prepared thereof for oral intake, such as in tablets.

The pharmaceutical preparations according to the invention may however be prepared for any route of administration, *e*.*g* oral, intravenous, cutaneous or subcutaneous, nasal, intramuscular or intraperitoneal. The nature of the carrier or other ingredients will depend on the specific route of administration. (Examples of techniques and protocols that are useful in this context are *inter alia* found in Remington's Pharmaceutical Sciences, 16^{th} edition. Osol, A (ed.), 1980.)

The dosage of these low molecular weight compounds will depend on the disease state or condition to be treted and other clinical factors such as weight and condition of the human or animal and the route of administration of the compound For treating human or animals. between approximately 0.5 mg/Kg of body weight to 500 mg/Kg of body weight of the compound can be administered.

The present compounds and methods are advantageously used in relation to all kind of angiogenesis related disorders and/or diseases, such as tumor conditions, diabetes, rheumatoid arthritis and even some inflammatory diseases, such as psoriasis, chronic inflammations of the intestines, asthma etc. It is also suggested that they may be used in order to treat and cure, or prevent, obesity.

In a particular embodiment, the present molecules are used in gene therapy. For a review of gene therapy procedures, see e.g. Anderson. *Science* (1992) 256:808-813.

A further advantageous use of the present invention is to develop methods of regulating the signalling of the present angiostatin and/or kringle 5 receptor in the body and therefore, the invention also relates to methods of treating a human or animal patient suffering from an angiogenesis related disease or disorder as well as to methods preventing such conditions.

### EXPERIMENTAL

Below, the invention will be disclosed in more detail with reference to the drawings.

All references in the present disclosure and above are hereby incorporated in the present application.

### Evidence that angiostatin binds to endothelial cells

By competitive binding assays between angiostatin and the angiogenic factors. VEGF and bFGF, the present inventors have shown that angiostatin does not affect the ligand-receptor interaction of these molecules. The same level of binding could be detected in the presence or absence of unlabeled angiostatin. Thus, it can be ruled out that angiostatin acts by blocking angiogenic factors interaction with endothelial cells.

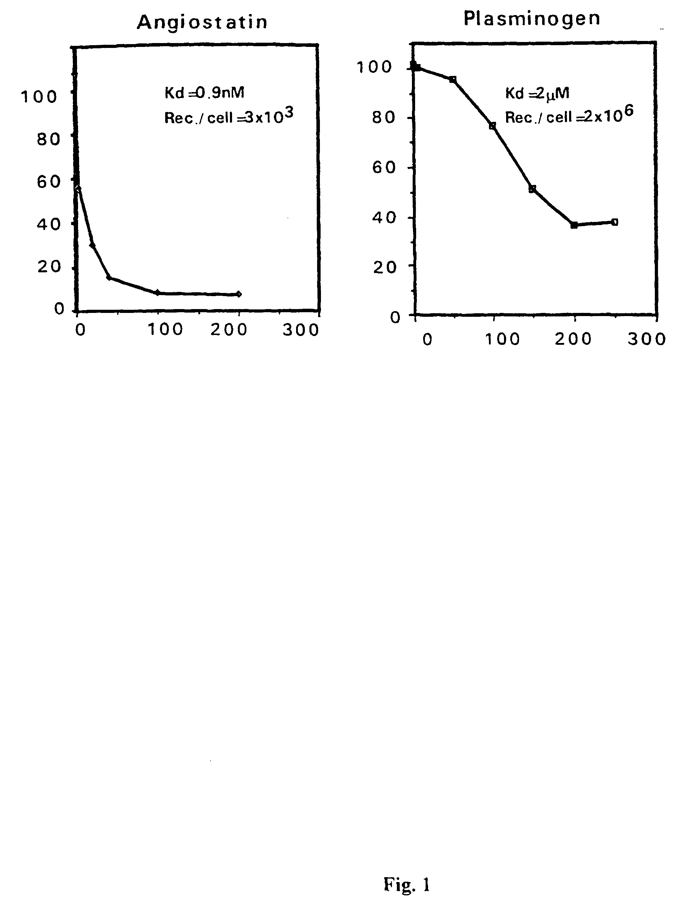

The direct binding of angiostatin and its precursor plasminogen has been studied by binding assays using iodinated proteins and analyzing their interaction with bovine microcapillary endothelial cells, see Figure 1.

In particular, human angiostatin (kringle domains 1-4) or plasminogen was labeled with iodine 125 by the Iodogen method according to the protocol of the manufacturer (Pierce Inc.). The labelled protein was then purified on a G50 sepharose column (Pharmacia Inc.) The specific activity was estimated at 90 000 cpm/ng protein. For binding assays, bovine capillary endothelial cells were grown to confluency in 12 well plates. The cells were washed with PBS containing 1mg/ml bovine serum albumine (BSA). The cells were then incubated with 10ng/ml radio-labeled angiostatin or plasminogen. The binding was competed with increasing concentrations of unlabeled ligand. Cells were incubated on ice for 2 hours. Cells were then washed five times with PBS+1mg/ml BSA. The cells were lysed with 1% Triton X100 in PBS and radioactivity was measured in a gamma counter. Dissociation constant and receptor numbers were estimated using the RBINDING program (van Zoelen EJ. Anal. Biochem. 1992 Feb 1:200 (2):393-9).

### Identification of angiostatin binding molecules using the yeast two hybrid system

Angiostatin retains activity even after reduction, suggesting that three dimensional folding is not vital for binding and activity. This favours screening procedures wherein a large number of clones may be screened although protein refolding may be less accurate.

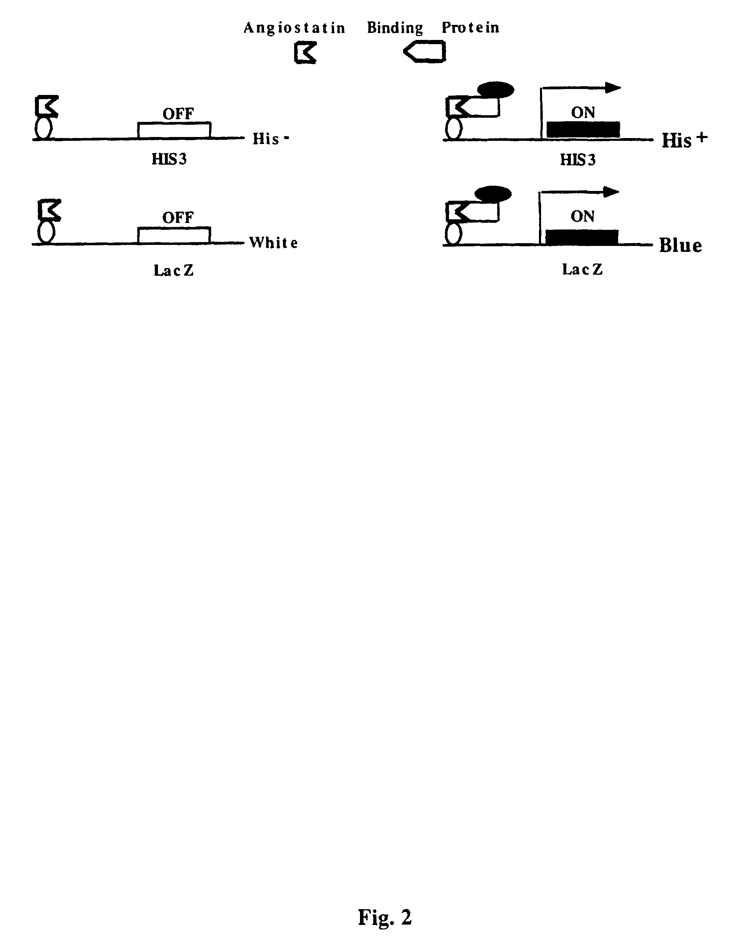

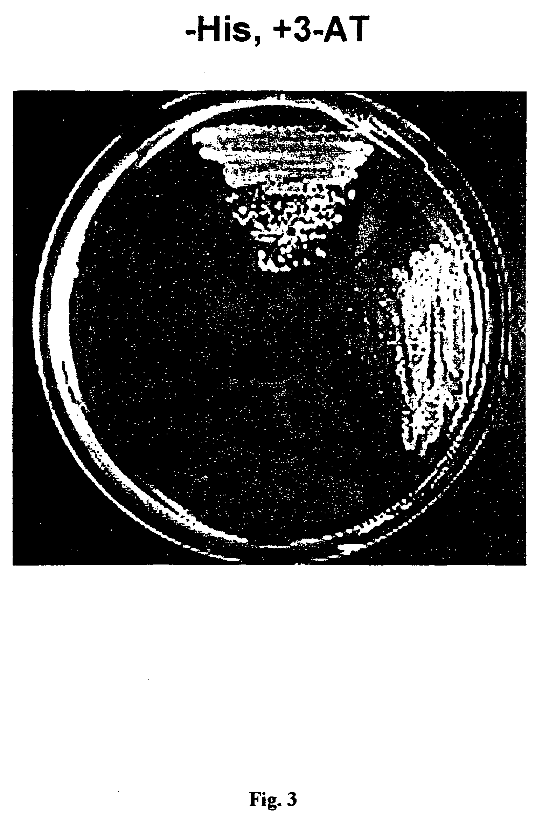

The yeast two hybrid system was employed to screen for molecules that bind kringle domains 1-4 of plasminogen. This is further disclosed below with reference to figure 2. Approximately 2x10⁶ clones from a two hybrid cDNA library from human term placenta (Stratagene) were screened. Positives clones were identified in the following way:
(1) Screening under selective conditions (His-, Leu- and Trp-) generated 37 positive clones in yeast strain CG1945.
(2) Seven out of 37 clones displayed high β-galactosidase activity after incubation with ONPG (SIGMA) at 30°C for 2h.
(3) The DNA from the seven colonies that contained high β-gal activity was purified and retransfected into yeast strain Y190. Three out of the seven colonies retained activity in the new yeast strain. Sequencing analysis of these clones revealed that they were derived from the same gene.
(4) Growth in the presence of 50mM 3-aminotriazol (Fig. 3) as well as β-gal activity (see table below) was assessed in Big 3 clones and compared to positive and negative controls.

| Binding Domain | Activation Domain | β-Gal Activity |
|---|---|---|
| Angiostatin + Binding Domain | Big3 - Activation Domain | 15 u |
| Angiostatin + Binding Domain | Activation Domain alone | <0.04 u |
| Binding Domain alone | Big3 - Activation Domain | 0.07 u |
| p53 + Binding Domain | Big3 - Activation Domain | 0.05 u |
| p53 + Binding Domain | SV40LT - Activation Domain | 90 u |

### Sequence of Big 3 (angiostatin-binding domain)

After isolation of the big 3 clone from a placenta yeast two-hybrid library (Stratagene, Inc.), we directly sequenced the cDNA insert from the pGAD activation domain vector with GAL4 AD sequencing primers. The cDNA insert was recloned into pUC18 vector using EcoR1 sites from adaptors and sequencing was repeated using universal and reverse primers. Sequences were analyzed in an ALF automated sequencer (Pharmacia). The sequence of Big 3 is shown in SEQ ID NO. 4.

### Expression and purification of Big3 and Big3-GST fusion protein

Big3 sequence has been expressed in *E.coli* as fusion protein with the glutathrone S - transferase (GST) domain from *Schistosoma japonicum*.

### • Vector construction:

Big3 fragment (429 bp) was obtained by PCR amplification using pUC18-Big3 plasmid as template. Primer sequences were:

The cycling protocol used was:

| | |
|---|---|
| 94°C 3', 60°C 1', 72°C 1' | 1 cycle |
| 94°C 30", 60°C 1'. 72°C 2' | 30 cycles |
| 94°C 30", 60°C 1', 72°C 5' | 1 cycle |

Tag polymerase: native Pfu DNA polymerase (Stratagene)

PCR fragment was digested with Bam*HI* and Xho*l*, purified and ligated to PGEX-6P-2 plasmid (Pharmacia Biotech) digested with the same restriction enzymes. Ligation mixture was used to transform XL1-Blue cells (Stratagene). Recombinant plasmids were verified by restriction analysis and automated sequencing.

### •Expression and purification:

DH5α cells (Clontech) were transformed with one verified clone, named pGEX-Big3, and induced for 6 hours, room temperature with 0.1 mM IPTG.

The fermentation broth was centrifuged at 4000xrpm 15 min and the cell pellet was resuspended in 10% w/v of lysis buffer (50 mM TRIS.HCl pH 8.0, 100 mM NaCl, 20 mM DTT , 1 mM EDTA, protease inhibitor mix), and lysed by sonication. The total lysate was centrifuged at 15000 xg for 20 min at 4°C. The clarified supernatant was applied to a Glutathione-Sepharose column (1ml for 20 ml of lysate) preequilibrated with lysis buffer. The resin was washed with 10 column volumes (CV) of lysis buffer and the fusion protein was eluted with 3 CV of elution buffer (100 mM TRIS.HCl pH 8.0 20 mM reduced glutathione).

GST-Big3 protein can be cleaved by incubation of the glutathione-Sepharose bound fusion protein with 20 ul/ml resin of PreScission protease in PS buffer containing 50 mM TRIS.HCl pH 7.0, 100 mM NaCl, 1 mM EDTA, 1 mM DTT, 1 mM PMSF.

The eluted proteins behave like a single peak in rp-HPLC (10-90% gradient of acetonitrile in water plus 0,1% TFA), show the expected molecular size by mass analysis (electrospray) and the correct NH2 sequence. In SDS-PAGE a contaminant appears at the apparent MW of 80 kD; it was demonstrated to be DnaK, a bacterial chaperone, which can be eliminated running an ion exchange chromatography on a HiTrapQ column.

The yields obtained from 1 litre of fermentation are about 10 mg of GST-Big3 fusion protein and about 3 mg of the cleaved Big3.

### In vitro binding of recombinant GST-tagged Big 3 to angiostatin

The protocol used for binding is described here below.

One colony of pGEX-Big3 transformant was inoculated into 10 ml LB medium plus ampicillin and grown overnight at 37°C.

The overnight culture was diluted 1:10 into 100 ml fresh medium and after 1 hour of incubation at 37°C, IPTG was added to a final concentration of 0.5mM and incubation continued for 3 to 7 hours.

The culture was then centrifuged at 5000g for 10 minutes, the pellet was resuspended in 3-5 ml of ice-cold PBS and cells were lysed by sonication. Triton X100 was added to a final concentration of 0.5 - 1 % and 1.5 ml aliquots of the suspension were centrifuged at 14000 rpm for10 minutes.

0.1-0.3ml of 50% slurry of glutathione-agarose.beads were added to the supernatant and mixed for 3-5 hour at 4°C. The beads were then collected by centrifugation and washed 5 times with ice-cold PBS and 2 times with binding solution (B.S.) consisting of: 50mM Tris pH7.5. 150mM NaCl. 0.5mM EDTA, 1mM DTT, 1mM PMSF).

Binding assay: 0.7ml B.S., 0.1% calf serum, 500ng angiostatin were mixed with 30-50µl 50% slurry of Big3-immobilized glutathione-agarose beads for 1 - 3 hour at 4°C.

The resin was washed 5-7 times with ice cold B.S., then used for Western blot analysis using anti-human plasminogen antibodies (Dako Inc.).

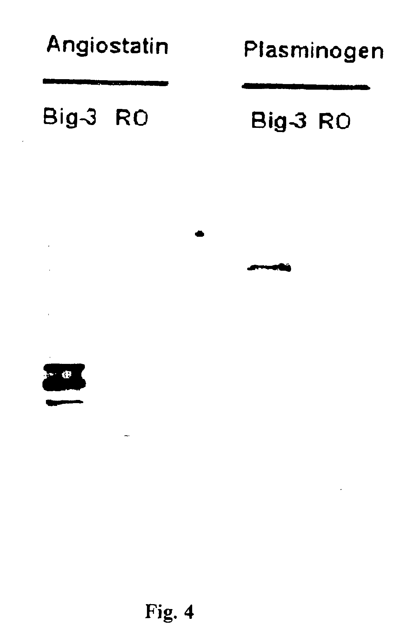

Figure 4 shows how purified recombinant Big 3 binds angiostatin in vitro. Interestingly, binding of plasminogen could also be detected, but only after reduction of disulphide bonds by treating it with DTT.

### Cloning of full-length sequence

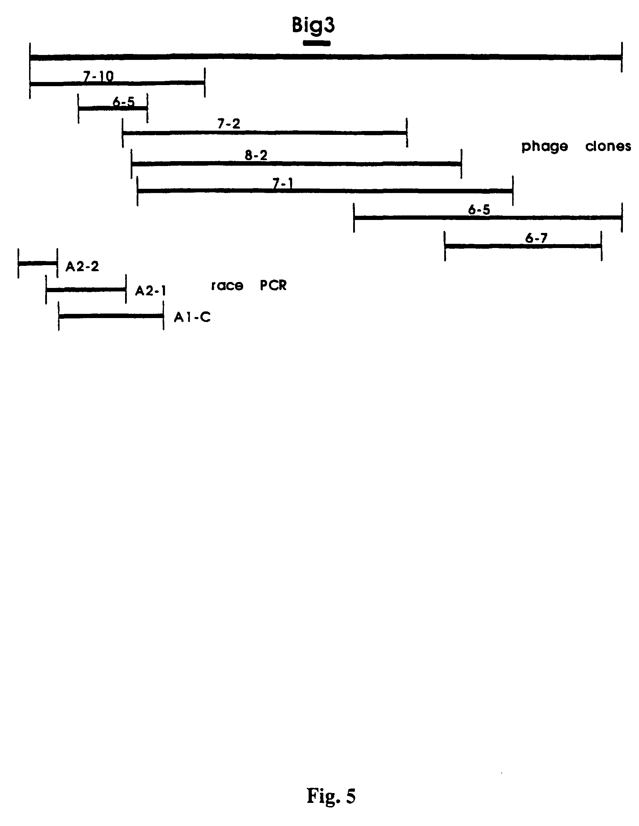

Figure 5 illustrates the scheme for cloning the full-length sequence of ABP-1. The whole gene was cloned by screening a placenta cDNA phage library with a Big3 probe (with repetitive sequences removed) together with 5' RACE PCR (Gibco) using mRNA from human umbilical cord endothelial cells. The full sequence of the cDNA clone is disclosed in SEQ ID NO. 1, whereas the encoded protein is shown in SEQ ID NO. 2. Details of the experimental protocol are as follows.
10 million clones of a placenta lambda phage library (Stratagene. Inc.) were screened using a Hin*fI* fragment of Big 3 as probe. DNA isolation and Southern blot procedures were performed according to established protocols (Sambrook et al 1989). We isolated clones (7-1, 7-2, 8-2, 9-3, 9-5) with sequences that overlap with Big3. The 5'-sequence of the 7-2 clone was used for designing Gene-Specific Primers (GSP) for 5'RACE PCR (Gibco Life technologies, Inc.). mRNA isolated from Human umbilical cord endothelial cells was used to identify 5' sequences.
Primers used for the first RACE PCR: RACE procedures have been used for amplification and cloning unknown sequences between the GSP2 and GSP3 and the 5'-end of the mRNA ABP-1. This sequence was then used to design new primers for the next set of RACE RCR:

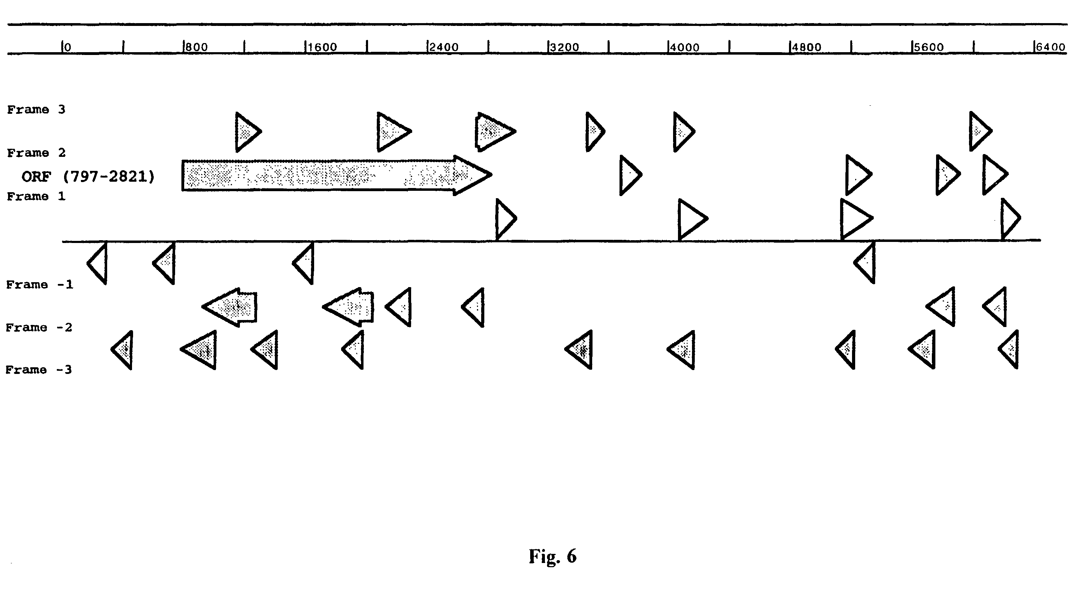

Clones A2-2, A2-1 and A1-C with overlapping sequences were isolated. The A2-1 sequence was used as probe for the second screening of the placenta phase library . From this screening we isolated another 2 clones ( 7-10 and 6-5). As shown in Figure 6, of the six possible frame the only one producing a complete open reading frame is frame 2, yielding a putative protein of 675 amino acid residues.

### mRNA Expression pattern

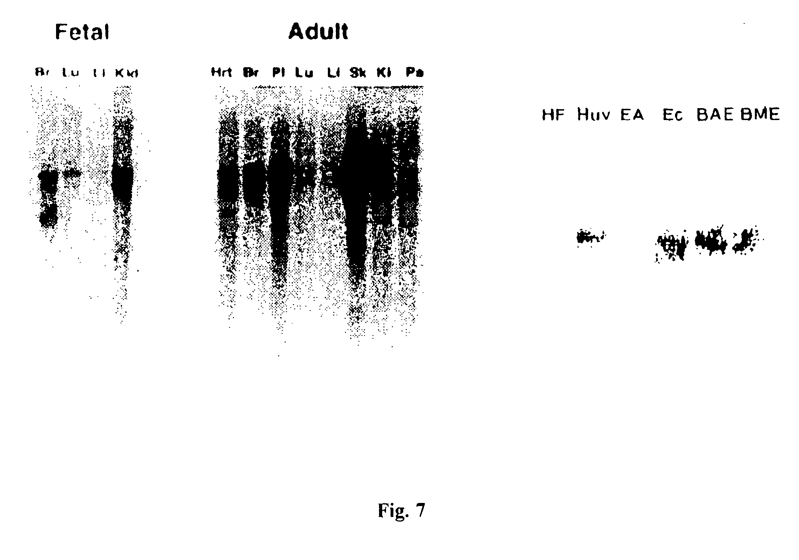

A commercially (Clontech, Inc.) obtained multiple human adult and fetal tissue (#7760-1 and #7756-1) mRNA blots (2mg mRNA/well) was probed for ABP-1. The blots were hybridized with the ExpressHyb hybridization solution (Clontech. Inc.) according to the protocol of the manufacturer. The blots were probed with the 7-2 5'Pst/ fragment. Figure 7 shows the results of the experiment; sizes are 9.5 and 7.5 kb.

ABP-1 was also detected in human umbilical cord, bovine aortic and bovine microcapillary endothelial cells. Little or no expression was detected in the immortalized endothelial cell-line EaHy926 and in human fetal fibroblasts. Neither of the cell lines respond to angiostatin or exhibit any binding of FITC-labeled angiostatin.

### Real time RT-PCR

The 5' nuclease assay (TaqMan assay) uses a nonextendable oligonucleotide hybridization probe (TaqMan Probe). The TaqMan probe consists of an oligonucleotide with a 5'-reporter dye and a 3'-quencher dye. When the probe is intact, the proximity of the reporter dye to the quencher dye results in suppression of the reporter fluorescence. primarily by a Foster-type energy transfer. During PCR, forward and reverse primers hybridize to a specific sequence of the target DNA. The TaqMan probe hybridizes to a target sequence within the PCR product. The Taq Polymerase cleaves the TaqMan probe with its 5'-3' nuclease activity. The reporter dye and quencher dye are separated upon cleavage, resulting in increased fluorescence of the reporter.

### Real time RT-PCR analysis

### RNA Preparation

Isolation and purification of total RNA from tissues and cells listed below was performed using the Ultraspec RNA isolation system (Biotex).

| HDMEC/2 | h.dermal microvascular EC | poly A+ |
|---|---|---|
| A2780 | h.ovarian carcinoma | TOTAL |
| A375 | h. melanoma | TOTAL |
| HDF | H.dermal fibroblasts | TOTAL |
| HELA | h.cervix carcinoma | TOTAL |
| DU145 | h.prostate carcinoma | TOTAL |
| HUVEC | h.umbelical vein EC | TOTAL |
| ECV304 | immortalized EC | TOTAL |
| CEM | h.acutelymphobl.leuk. | TOTAL |
| K562 | h.erythroleukemia | TOTAL |
| JURKAT | h.acuteT-cellleukcmia | TOTAL |
| THP-1 | h.monocyte(acute leu) | TOTAL |
| EaHy926 | h.dermal cells | TOTAL |
| S35/K9 | human colon cancer | TOTAL |
| Brain | human normal tissue | TOTAL |
| Colon | human normal tissue | TOTAL |
| Prostate | human normal tissue | TOTAL |
| Skel.Muscle | human normal tissue | TOTAL |
| Uterus | human normal tissue | TOTAL |
| Placenta | human normal tissue | TOTAL |

### cDNA Synthesis

The RT reaction was performed using TaqMan Reverse Transcription Reagent (PE Applied Biosystems) with Random hexamers, as RT primers. The reaction volume for Reverse Transcription step was 100 µl and the total RNA amount was 1 µg for sample.

The RT was performed using the following cycling parameter:
10' at 25°C
45' at 48°C
5' at 95°C

### PCR reaction

After a primer concentration optimisation, the PCR reaction on 10 ng of the cDNA was performed using TaqMan Universal PCR Master Mix (PE Applied Biosystems) and the following oligonucleotides
- ABP-1 Forward Primer: 5' GTTTGACCTGCAATCCAGACAA 3' (SEQ ID NO. 7) Final concentration 300nM
- ABP-1 Reverse Primer: 5' CCCAGGATCTGAATGGGAGTT 3' (SEQ ID NO. 8) Final concentration 900nm
- ABP-1 TaqMan Probe:
   5' (FAM dye)-CAGATGGGCCTGTGTTCCACTCCAA-(TAMRA dye) 3' (SEQ ID NO. 9) Final probe concentration 200nM

The cycling protocols was:
2' at 50°C; 10' at 95°C 1 cycle
15'' at 95°C; 1' at 60°C 40 cycles

### Relative Quantitation of gene expression

The Comparative method use an arithmetic formula to achieve the result for relative quantitation without need for Standard curve.

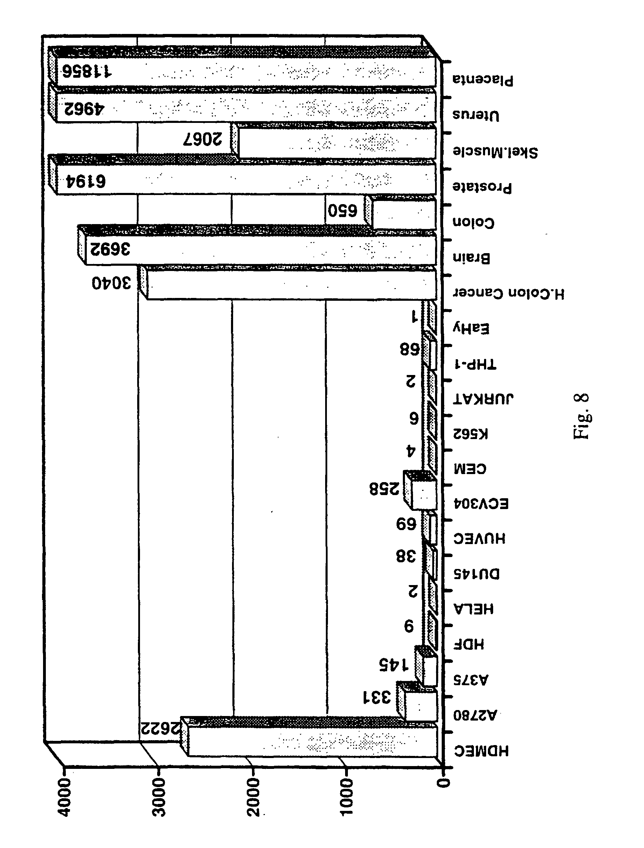

The result , depicted in Figure 8, indicate how many fold the sample X expresses the target relative to the Calibrator which is the sample that shows the lowest level of expression of the target. In this experiment EaHY936 sample was chosen as calibrator.

### Antibodies against Big-3

For immunisation of New Zealand White rabbits, 100 micrograms of Big3-GST fusion protein were dissolved in 1 ml phosphate buffered saline (PBS) homogenised with 1 ml of Freund's Complete Adjuvant (Gibco). The resulting emulsion was injected subcutaneously on day 0 and this treatment was repeated on days 15 and 28. Blood was removed from rabbits on day 35, allowed to coagulate overnight at 4 °C. and the resulting serum stored at -20°C.

For purification of specific antibodies. resin-immobilised ligand was produced as follows: Big3 polypeptide was diluted in 0.1M sodium bicarbonate, 0.5 M NaCl, pH8.3, at a concentration of 5 mg/ml in a total volume of 2 ml. This was reacted for two hours at room temperature with 2 ml of cyanogen bromide-activated Sepaharose CL-4B resin (Sigma Chemical Co. St Louis, MO). After reaction, the resin was washed three times in 10 volumes of 100 mM Tris. 500 mM NaCl, pH 7.5. Immune serum was incubated with the affinity resin for two hours at 4°C, after which resin was washed in a 5 ml glass chromatography column (Bio-Rad, Richmond CA)for with 25 volumes of 100 mM Tris, 500 mM NaCl, pH 7.5. Specific antibodies were eluted in 1 ml aliquots with 100 mM Glycine/HCl, pH 2.8. Elution of antibody was followed by monitoring the optical density at 280 nM (OD280), and fractions with an OD280 of greater than 1 were pooled and dialysed (Slide-A-lyzer cassettes, Pierce) for 36 hours at 4°C against 1 liter PBS, with three changes of buffer.

### Binding and signalling of angiostatin via the ABP-1 protein

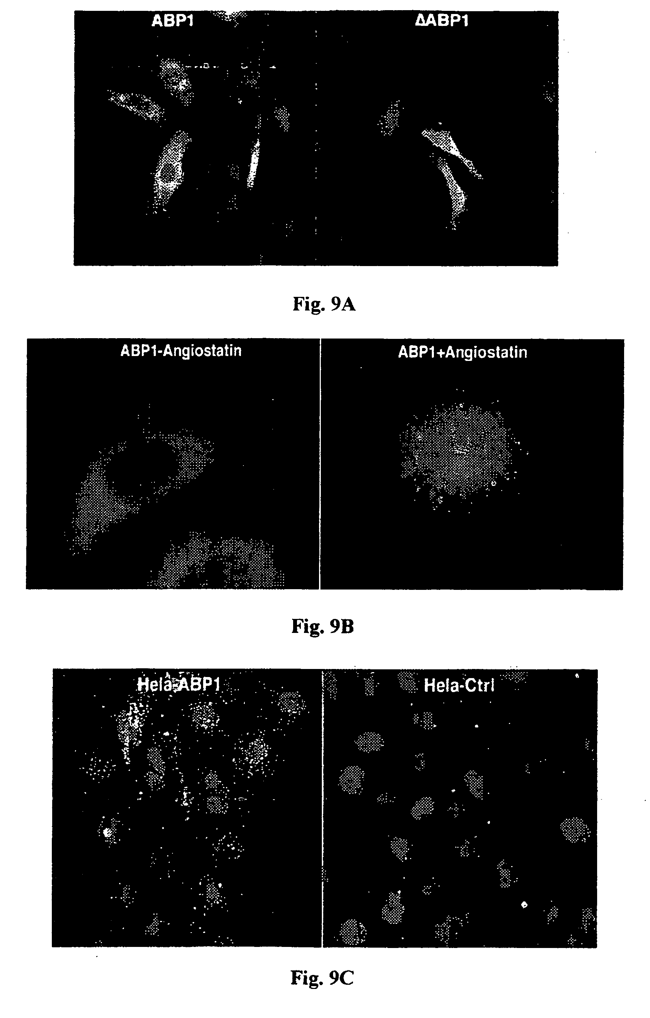

Figures 9A and 9B show data of binding of FITC-labeled angiostatin to ABP-1 transfected HeLa and EaHy926 cells transfected with the vector control. In particular figure 9A shows the cellular localization of ABP-1 fused to green fluorescent protein (GFP) in transiently transfected cells. The protein can be detected in the endoplasmic reticulum and the cell membrane. DABP-1 contains a 500bp deletion in the 5'end of the gene, which disrupts the previously described localization of ABP-1.

Figure 9B shows HeLa cells transfected with ABP-1 GFP. Incubation with 2.5 mg of angiostatin for 60 minutes at 0°C and subsequent incubation for 15 minutes at 37°C induces internalization of ABP-1-GFP.

Figure 9C shows the binding of angiostatin to ABP-1. We have shown that fluorescein isothiocyanate (FITC)-labelled angiostatin binds specifically to endothelial cells (data not shown). We have transfected HeLa cells with either the ABP-1 expression construct or the vector control (p RC/CMV. In Vitrogen. Inc.). We incubated live HeLa cells FITC-labelled angiostatin (10ug/ml) in DMEM+10% fetal calf serum at 0°C for 60 minutes. The cells were then incubated at 37°C at 15 minutes to aggregate hound angiostatin. Binding was analyzed with a fluorescent microscope. Binding of angiostatin was detected in ABP-1 transfected cells but not in the vector control.

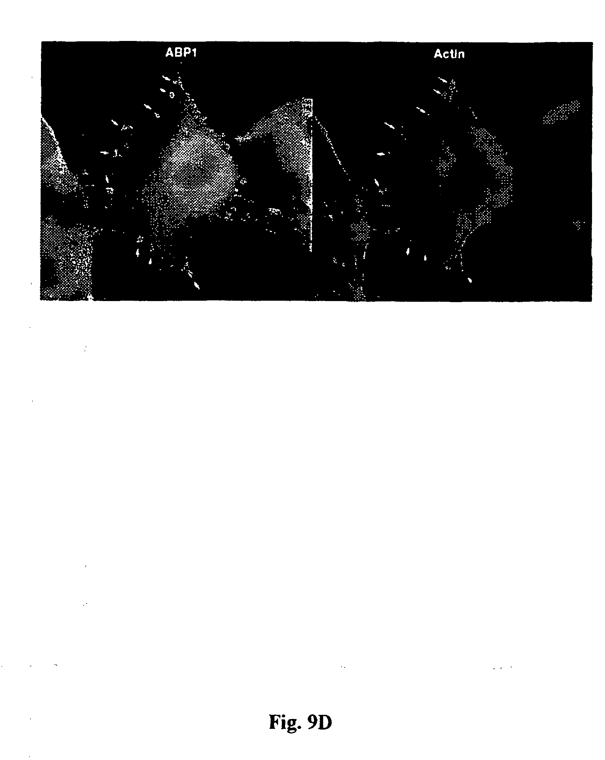

Figure 9D shows immunostaining of ABP-1 in Human umbilical cord endothelial (HUVE) cells together with staining against F-actin with rhodamin-labelled phalloidin.

### •Immunostaining protocol:

HUVE cells were fixed in 4 % formaldehyde, washed in PBS and preblocked in 5% horse serum. The blocking solution was removed and replaced by rabbit polyclonal antibodies against the angiostatin-binding domain Big3 diluted in 5% horse serum. Positive staining was visualized with a fluorescence-labeled secondary antibody (Dako, Inc.). Rhodamin-conjugated phalloidin (Molecular Probes. Inc.) was stained simultaneously with the secondary antibody (after permeabilization with 1%triton X100 for 1 minute). Rabbit polyclonal antibodies against green fluorescent protein was used as a negative control. In addition no positive staining could be detected in human fibroblasts.

As can be seen in Fig. 9D. ABP-1 is localized in focal adhesions and membrane ruffles (arrows).

### In vitro kinase data

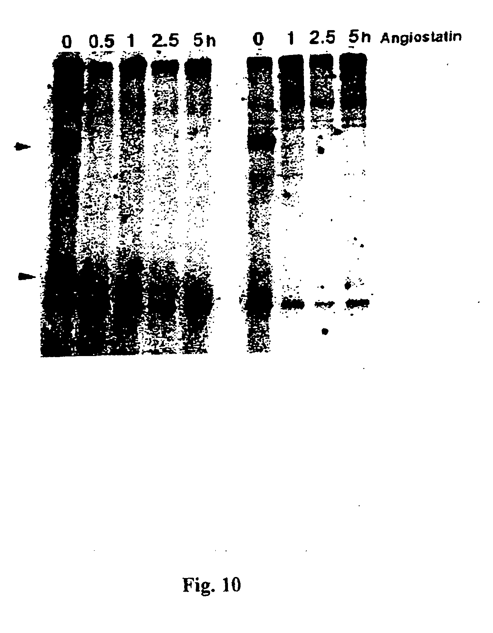

Huve cells were plated subconfluent in 5cm Petri dishes. Angiostatin was added at 2.5 mg/ml at different time points the following day. All plates including controls were harvested simultanously. The cells were rinsed in ice cold PBS and incubated in 1ml lysis buffer. The cells were transferred to an eppendorf tube and centrifuged at 14 000rpm for 5 minutes at 4°C. The supernatant was transferred to another eppendorf tube and 2µg of Big 3 rabbit polyclonal antibodies was added. The samples were incubated for 60 min. at 4°C and subsequently 50µl of a protein A sepharose (Pharmacia, Inc.) slurry was added and incubated for another 60 min. in a rotating incubator. The immunoprecipitates were collected by brief centrifugation and washed two times in lysis buffer, once in washing buffer and once in kinase buffer. Residual buffer was removed with a syringe, 25 µl of kinase buffer was added to each tube together with 1µCi gammaATP (Amersham. Inc.). The samples were incubated at R.T. for 20 minutes. The reaction was stopped by adding 2xSDS PAGE sample buffer. The samples were boiled under denaturing conditions and subsequently analyzed by SDS PAGE.
The data illustrated in Figure 10 show that addition of angiostatin downregulates ABP-1 associated kinase activity within 30 minutes. This is direct proof that angiostatin affects signaling pathways that are mediated by ABP-1.

### ABP-1 mediates Angiostatin-induced focal adhesion kinase activity.

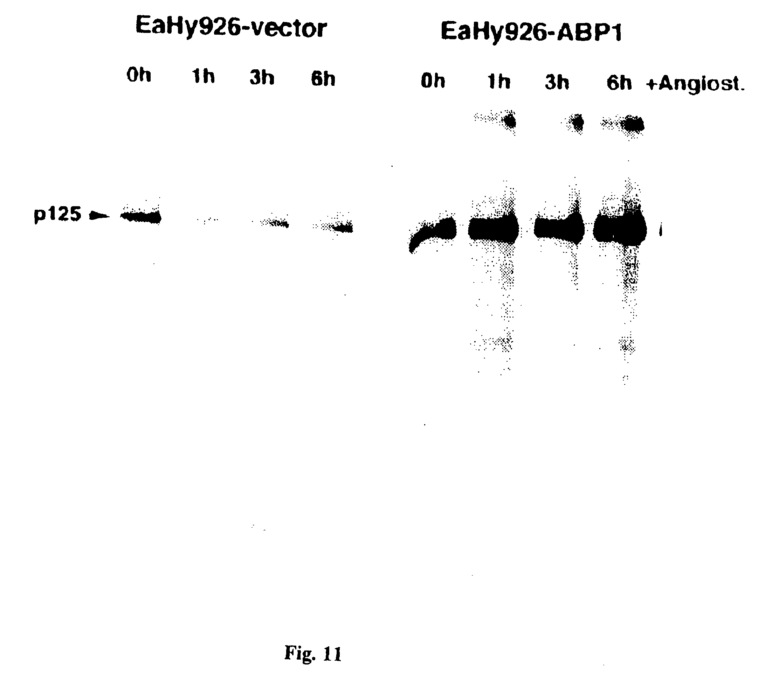

The data illustrated by Figure 11 show that addition of 2.5 µg/ml of angiostatin upregulates FAK activity within 1 hour after addition.

EaHy926 cells were plated subconfluent in 5cm Petri dishes. Angiostatin was added at 2.5 µg/ml at different time points the following day. All plates including controls were harvested simultaneously. The cells were rinsed in ice cold PBS and incubated in 1ml lysis buffer. The cells were transferred to an eppendorf tube and centrifuged at 14 000rpm for 5 minutes at 4°C. The supernatant was transferred to another eppendorf tube and 1ug of FAK monoclonal antibody (Transduction Lab. Inc.) was added. The samples were incubated for 60 min. at 4°C and subsequently rabbit anti-mouse IgG + 50µl of a protein A sepharose (Pharmacia, Inc.) slurry was added and incubated for another 60 min. in a rotating incubator. The immunoprecipitates were collected by brief centrifugation and washed two times in lysis buffer, once in washing buffer and once in kinase buffer. Residual buffer was removed with a syringe. 25 µl of kinase buffer was added to each tube together with 1µCi γATP (Amersham. Inc.). The samples were incubated at R.T. for 20 minutes. The reaction was stopped by adding 2xSDS PAGE sample buffer. The samples were boiled under denaturing conditions and subsequently analyzed by SDS PAGE.

As can be seen in Fig. 11, addition of angiostatin upregulates FAK activity within 1 hour. It should be noted that EaHy926 cells are not expressing ABP1 as demonstrated by reverse transcriptase PCR analysis.

## Claims

1. An isolated mammal angiostatin-binding protein-1 (ABP-1) capable of binding the kringle domains 1 to 4 and/or 5 of plasminogen and comprising an amino acid sequence having a sequence homology greater than at least 80% to SEQ ID NOs. 2, 3 or 4.

2. The protein of claim 1, wherein the protein is a human protein.

3. The protein of claim 1 or 2, wherein the amino acid sequence has at least 90 % sequence homology to SEQ ID NOs. 2, 3 or 4.

4. The protein of claim 1 or 2, wherein the amino acid sequence has at least 95 % sequence homology to SEQ ID NOs. 2, 3 or 4.

5. The protein of claim 1 or 2, wherein the amino acid sequence has at least 98 % sequence homology to SEQ ID NOs. 2, 3 or 4.

6. The protein of claim 5, which comprises the amino acid sequence of SEQ ID NO. 2.

7. The protein of claim 5, which comprises the amino acid sequence of SEQ ID NO. 3, wherein the amino acid residue in position 135 is Asn, Ser or Asp and the three amino acid residues in positions 148 to 150 are the tripeptide Glu-Leu-Ala or the tripeptide Thr-Trp-Pro.

8. The protein of claim 5, which comprises the amino acid sequence of SEQ ID NO. 4.

9. An isolated nucleic acid molecule comprising a sequence that codes for a protein or peptide according to any one of claims 1 to 8.

10. The isolated nucleic acid molecule of claim 9 encoding the protein of claim 6.

11. The isolated nucleic acid molecule of claim 9 encoding the protein of claim 7.

12. The isolated nucleic acid molecule of claim 9 encoding the protein of claim 8.

13. The isolated nucleic acid molecule of any one of claims 10 to 12 comprising at least the sequence from nucleotide 2177 to nucleotide 2608 of SEQ ID NO. 1.

14. The isolated nucleic acid molecule of claim 9 comprising the sequence from nucleotide 797 to nucleotide 2824 of SEQ ID NO. 1.

15. The isolated nucleic acid molecule of claim 14 consisting of the sequence of SEQ ID NO.1.

16. A vector comprising a nucleic acid according to any one of claims 9 to 15.

17. The vector of claim 16, which is a plasmid.

18. The vector of claim 16, which is a virus.

19. A recombinant cell transformed or transfected with the vector of any one of claims 16-18.

20. An antibody or antibody fragment, which specifically binds a protein according to any one of claims 1 to 8.

21. An antibody according to claim 20, which is a monoclonal antibody or fragment thereof.

22. The antibody of claims 20 or 21 for use as a medicament.

23. A recombinant cell expressing the antibody according to claims 20 or 21.

24. A screening method for identifying a compound capable of interacting with a protein according to any one of claims 1 to 8.

25. The proteins, molecules, antibodies or antibody fragments of any one of claims 1 to 8, 9-15 or 20-21 for use as a medicament.

26. Use of the proteins, molecules, antibodies or antibody fragments of any one of claims 1 to 8, 9-15 or 20-21 in the manufacture of a medicament directed towards an angiogenesis-related disease or disorder.

27. A pharmaceutical preparation comprising a protein, a molecule, an antibody or an antibody fragment of any one of claims 1 to 8, 9-15 or 20-21 together with a pharmaceutically acceptable carrier.

## Patentansprüche

1. Isoliertes Säugetier-Angiostatin-bindendes Protein-1 (ABP-1), das die Kringle-Domänen 1 bis 4 und/oder 5 von Plasminogen binden kann und eine Aminosäuresequenz mit einer Sequenzhomologie von mehr als zumindest 80 % mit mit SEQ ID NOs. 2, 3 oder 4 umfasst.

2. Protein gemäß Anspruch 1, worin das Protein ein humanes Protein ist.

3. Protein gemäß Anspruch 1 oder 2, worin die Aminosäuresequenz wenigstens 90 % Sequenzhomologie mit SEQ ID NOs. 2, 3 oder 4 aufweist.

4. Protein gemäß Anspruch 1 oder 2, worin die Aminosäuresequenz wenigstens 95 % Sequenzhomologie mit SEQ ID NOs. 2, 3 oder 4 aufweist.

5. Protein gemäß Anspruch 1 oder 2, worin die Aminosäuresequenz wenigstens 98 % Sequenzhomologie mit SEQ ID NOs. 2, 3 oder 4 aufweist.

6. Protein gemäß Anspruch 5, das die Aminosäuresequenz von SEQ ID NO. 2 umfasst.

7. Protein gemäß Anspruch 5, das die Aminosäuresequenz von SEQ ID NO. 3 umfasst, worin der Aminosäurerest in Position 135 Asn, Ser oder Asp ist und die drei Aminosäurereste in den Positionen 148 bis 150 das Tripeptid Glu-Leu-Ala oder das Tripeptid Thr-Trp-Pro sind.

8. Protein gemäß Anspruch 5, das die Aminosäuresequenz von SEQ ID NO. 4 umfasst.

9. Isoliertes Nukleinsäuremolekül, das eine Sequenz umfasst, die für ein Protein oder Peptid gemäß einem der Ansprüche 1 bis 8 codiert.

10. Isoliertes Nukleinsäuremolekül gemäß Anspruch 9, das das Protein gemäß Anspruch 6 codiert.

11. Isoliertes Nukleinsäuremolekül gemäß Anspruch 9, das das Protein gemäß Anspruch 7 codiert.

12. Isoliertes Nukleinsäuremolekül gemäß Anspruch 9, das das Protein gemäß Anspruch 8 codiert.

13. Isoliertes Nukleinsäuremolekül gemäß einem der Ansprüche 10 bis 12, das zumindest die Sequenz von Nukleotid 2177 bis Nukleotid 2608 von SEQ ID NO. 1 umfasst.

14. Isoliertes Nukleinsäuremolekül gemäß Anspruch 9, das die' Sequenz von Nukleotid 797 bis Nukleotid 2824 von SEQ ID NO. 1 umfasst.

15. Isoliertes Nukleinsäuremolekül gemäß Anspruch 14, das aus der Sequenz von SEQ ID NO. 1 besteht.

16. Vektor, der eine Nukleinsäure gemäß einem der Ansprüche 9 bis 15 umfasst.

17. Vektor gemäß Anspruch 16, der ein Plasmid ist.

18. Vektor gemäß Anspruch 16, der ein Virus ist.

19. Rekombinante Zelle, die mit dem Vektor gemäß einem der Ansprüche 16 bis 18 transformiert oder transfiziert ist.

20. Antikörper oder Antikörperfragment, der/das spezifisch ein Protein gemäß einem der Ansprüche 1 bis 8 bindet.

21. Antikörper gemäß Anspruch 20, der ein monoklonaler Antikörper oder ein Fragment davon ist.

22. Antikörper gemäß Anspruch 20 oder 21 zur Verwendung als Medikament.

23. Rekombinante Zelle, die den Antikörper gemäß Anspruch 20 oder 21 exprimiert.

24. Durchmusterungsverfahren zur Identifizierung einer Verbindung, die mit einem Protein gemäß einem der Ansprüche 1 bis 8 wechselwirken kann.

25. Proteine, Moleküle, Antikörper oder Antikörperfragmente gemäß einem der Ansprüche 1 bis 8, 9 bis 15 oder 20 bis 21 zur Verwendung als Medikament.

26. Verwendung der Proteine, Moleküle, Antikörper oder Antikörperfragmente gemäß einem der Ansprüche 1 bis 8, 9 bis 15 oder 20 bis 21 in der Herstellung eines Medikaments, das auf eine Angiogenese-bezogene Krankheit oder Störung gerichtet ist.

27. Pharmazeutische Zubereitung, die ein Protein, ein Molekül, einen Antikörper oder ein Antikörperfragment gemäß einem der Ansprüche 1 bis 8, 9 bis 15 oder 20 bis 21 zusammen mit einem pharmazeutisch akzeptablen Träger umfasst.

## Revendications

1. Protéine ABP-1 (protéine se liant à l'angiostatine) mammalienne isolée, capable de se lier aux domaines kringle 1 à 4 et/ou 5 du plasminogène et comportant une séquence d'acides aminés qui présente au moins plus de 80 % d'homologie avec l'une des séquences n° 2, 3 et 4.

2. Protéine conforme à la revendication 1, laquelle protéine est une protéine humaine.

3. Protéine conforme à la revendication 1 ou 2, dans laquelle la séquence d'acides aminés présente au moins 90 % d'homologie avec l'une des séquences n° 2, 3 et 4.

4. Protéine conforme à la revendication 1 ou 2, dans laquelle la séquence d'acides aminés présente au moins 95 % d'homologie avec l'une des séquences n° 2, 3 et 4.

5. Protéine conforme à la revendication 1 ou 2, dans laquelle la séquence d'acides aminés présente au moins 98 % d'homologie avec l'une des séquences n° 2, 3 et 4.

6. Protéine conforme à la revendication 5, qui comporte la séquence d'acides aminés présentée en tant que séquence n° 2.

7. Protéine conforme à la revendication 5, qui comporte la séquence d'acides aminés présentée en tant que séquence n° 3, dans laquelle le résidu d'acide aminé situé en position 135 est un résidu Asn, Ser ou Asp et les trois résidus d'acides aminés situés en positions 148 à 150 forment un tripeptide Glu-Leu-Ala ou Thr-Trp-Pro.

8. Protéine conforme à la revendication 5, qui comporte la séquence d'acides aminés présentée en tant que séquence n° 4.

9. Molécule d'acide nucléique isolée, comportant une séquence qui code une protéine ou un peptide conforme à l'une des revendications 1 à 8.

10. Molécule d'acide nucléique isolée, conforme à la revendication 9, qui code une protéine conforme à la revendication 6.

11. Molécule d'acide nucléique isolée, conforme à la revendication 9, qui code une protéine conforme à la revendication 7.

12. Molécule d'acide nucléique isolée, conforme à la revendication 9, qui code une protéine conforme à la revendication 8.

13. Molécule d'acide nucléique isolée, conforme à l'une des revendications 10 à 12, qui comporte au moins la séquence qui va du nucléotide 2177 au nucléotide 2608 de la séquence n° 1.

14. Molécule d'acide nucléique isolée, conforme à la revendication 9, qui comporte la séquence qui va du nucléotide 797 au nucléotide 2824 de la séquence n° 1.

15. Molécule d'acide nucléique isolée, conforme à la revendication 14, qui est constituée de la séquence n° 1.

16. Vecteur comportant un acide nucléique conforme à l'une des revendications 9 à 15.

17. Vecteur conforme à la revendication 16, qui est un plasmide.

18. Vecteur conforme à la revendication 16, qui est un virus.

19. Cellule recombinante, transformée ou transfectée avec un vecteur conforme à l'une des revendications 16 à 18.

20. Anticorps ou fragment d'anticorps, qui se lie spécifiquement à une protéine conforme à l'une des revendications 1 à 8.

21. Anticorps conforme à la revendication 20, qui est un anticorps monoclonal ou un fragment d'un tel anticorps.

22. Anticorps conforme à la revendication 20 ou 21, destiné à ser-vir de médicament.

23. Cellule recombinante exprimant un anticorps conforme à la revendication 20 ou 21.

24. Procédé de criblage ayant pour but d'identifier un composé capable d'interagir avec une protéine conforme à l'une des revendications 1 à 8.

25. Protéine, molécule, anticorps ou fragment d'anticorps, confor-me à l'une des revendications 1 à 8, 9 à 15 ou 20 et 21, destiné à servir de médicament.

26. Emploi d'une protéine, d'une molécule, ou d'un anticorps ou fragment d'anticorps, conforme à l'une des revendications 1 à 8, 9 à 15 ou 20 et 21, dans la fabrication d'un médicament contre un trouble ou une maladie en rapport avec l'angiogenèse.

27. Préparation pharmaceutique contenant une protéine, une molécule, ou un anticorps ou fragment d'anticorps, conforme à l'une des revendications 1 à 8, 9 à 15 ou 20 et 21, conjointement avec un véhicule pharmacologiquement admissible.


### SEQUENCE LISTING

<110> Pharmacia & Upjohn
<120> Angiogenesis related molecules
<130> pctpha1856
<140>
   <141>
<150> SE9804372-2
   <151> 1998-12-17
<160> 15
<170> PatentIn Ver. 2.1
<210> 1
   <211> 6463
   <212> DNA
   <213> Homo sapiens
<220>
   <221> CDS
   <222> (797)..(2824)
<400> 1
   ccaggagctg ccttggcagt cacgcccctt ccttccgagg agctttctgg ctgcctaaac 60
   tggtagaccc cctgaattac tcctccatct ccgctctctt tcgcctcctc ttctcttagt 120
   tctctccgcc tccccctcaa ctaccaccac ctccagtcag tctcgcctcc ggctatccgc 180
   tgctccaccc tctggcccgg tatcctgcct gtccgctgcc accaaggaga gcccggacgg 240
   agcagcgagg aggggagcag ccgggagttg gggcttcccc cctgcccatc cctggccgct 300
   ggcccgggac cgaagccact tgagcgagca gagagtcgtc accttgtctt ctttgccttc 360
   agggagctgc taagaaggac aaataagata gcagagtgaa agagcttttg tctccttaga 420
   aggaaggctg agaaactaaa ggccagcgca ggacatctca ttgccattgt cagccaggaa 480
   ctcgcagcct cacagcccta cttcttctct gacctctggg gggtccttgc ccttgctaca 540
   atctccacca tccactagat tgtctcctgc ccgacacccc ttggtcccaa accagggaga 600
   ccattcagct cacctgccta ggccgcagca gcatttcctt cctaatcagg ctcaccaggg 660
   ggatcattac cgtctctccc aacctggcct gagtcagcag cagcagcaac agcagcagca 720
   gcaccatcat caccatcacc accaacaaca gcagcagcag cagccacagc agcagccagg 780
   agaagcctat tcagct atg cct cgg gct cag cca tcc tct gct tct tat cag 832
                     Met Pro Arg Ala Gln Pro Ser Ser Ala Ser Tyr Gln
                     1               5                   10
   cca gtg cca gca gac cct ttt gcc att gtt tcc aga gcc cag cag atg   880
   Pro Val Pro Ala Asp Pro Phe Ala Ile Val Ser Arg Ala Gln Gln Met
           15                  20                  25
   gtt gag atc ctc tca gac gag aac cgg aac ttg agg caa gag ttg gaa   928
   Val Glu Ile Leu Ser Asp Glu Asn Arg Asn Leu Arg Gln Glu Leu Glu
       30                  35                  40
   gga tgc tat gag aag gtg gca aga ctg cag aag gtg gag aca gaa atc   976
   Gly Cys Tyr Glu Lys Val Ala Arg Leu Gln Lys Val Glu Thr Glu Ile
   45                  50                  55                  60
   cag cgc gtc tcg gag gca tat gag aac ctc gtg aag tca tcc tcc aaa   1024
   Gln Arg Val Ser Glu Ala Tyr Glu Asn Leu Val Lys Ser Ser Ser Lys
                   65                  70                  75
   aga gag gcc cta gag aaa gcc atg aga aac aag cta gag ggc gag att   1072
   Arg Glu Ala Leu Glu Lys Ala Met Arg Asn Lys Leu Glu Gly Glu Ile
               80                  85                  90
   cgg agg atg cat gat ttc aac agg gat ctg aga gag cgt cta gag act   1120
   Arg Arg Met His Asp Phe Asn Arg Asp Leu Arg Glu Arg Leu Glu Thr
           95                  100                 105
   gcc aac aag cag ctt gca gag aag gaa tat gag ggg tca gag gac acc   1168
   Ala Asn Lys Gln Leu Ala Glu Lys Glu Tyr Glu Gly Ser Glu Asp Thr
       110                 115                 120
   aga aaa acc atc tcg cag ctc ttt gca aaa aat aaa gaa agc cag cgt   1216
   Arg Lys Thr Ile Ser Gln Leu Phe Ala Lys Asn Lys Glu Ser Gln Arg
   125                 130                 135                 140
   gag aag gag aag ctg gaa gcg gag ctg gcc act gcc cgt tct acc aat   1264
   Glu Lys Glu Lys Leu Glu Ala Glu Leu Ala Thr Ala Arg Ser Thr Asn
                   145                 150                 155
   gag gac caa aga cga cac atc gaa atc cga gat cag gcc ctg agt aat   1312
   Glu Asp Gln Arg Arg His Ile Glu Ile Arg Asp Gln Ala Leu Ser Asn
               160                 165                 170
   gcc cag gcc aag gtg gta aag ctg gaa gaa gag ctg aaa aag aag caa   1360
   Ala Gln Ala Lys Val Val Lys Leu Glu Glu Glu Leu Lys Lys Lys Gln
           175                 180                 185
   gtg tac gtt gac aag gtg gag aag atg cag cag gcc ctt gta cag ctc 1408
   Val Tyr Val Asp Lys Val Glu Lys Met Gln Gln Ala Leu Val Gln Leu
   190 195 200
   cag gca gca tgt gaa aaa cgt gag cag cta gag cac cgt ctc cgg aca 1456
   Gln Ala Ala Cys Glu Lys Arg Glu Gln Leu Glu His Arg Leu Arg Thr
   205 210 215 220
   cga ctg gag agg gaa ctg gaa tcc ctg aga atc cag cag cgt cag ggc 1504
   Arg Leu Glu Arg Glu Leu Glu Ser Leu Arg Ile Gln Gln Arg Gln Gly
   225 230 235
   aac tgt cag ccc acc aac gtt tca gaa tac aat gct gcc gca ctg atg 1552
   Asn Cys Gln Pro Thr Asn Val Ser Glu Tyr Asn Ala Ala Ala Leu Met
   240 245 250
   gag ctc ctt cgg gag aaa gag gag agg att ctg gct ctg gaa gct gat 1600
   Glu Leu Leu Arg Glu Lys Glu Glu Arg Ile Leu Ala Leu Glu Ala Asp
   255 260 265
   atg aca aag tgg gag cag aaa tat ttg gag gag aat gtg atg aga cat 1648
   Met Thr Lys Trp Glu Gln Lys Tyr Leu Glu Glu Asn Val Met Arg His
   270 275 280
   ttt gct ctg gat gct gct gca act gtg gct gct cag agg gac aca aca 1696
   Phe Ala Leu Asp Ala Ala Ala Thr Val Ala Ala Gln Arg Asp Thr Thr
   285 290 295 300
   gtc atc agt cac tct cct aac acc agc tat gac aca gct cta gaa gct 1744
   Val Ile Ser His Ser Pro Asn Thr Ser Tyr Asp Thr Ala Leu Glu Ala
   305 310 315
   cgc atc cag aaa gag gag gaa gaa atc ttg atg gcc aat aag cgt tgc 1792
   Arg Ile Gln Lys Glu Glu Glu Glu Ile Leu Met Ala Asn Lys Arg Cys
   320 325 330
   ctt gac atg gag ggc agg att aag acc ctc cat gcc cag att att gag 1840
   Leu Asp Met Glu Gly Arg Ile Lys Thr Leu His Ala Gln Ile Ile Glu
   335 340 345
   aag gat gcc atg atc aaa gta ctc cag cag cgt tcc cgg aag gag ccg 1888
   Lys Asp Ala Met Ile Lys Val Leu Gln Gln Arg Ser Arg Lys Glu Pro
   350 355 360
   agc aag aca gag cag ctg tcg tgc atg cgg cca gcg aag tct ctg atg 1936
   Ser Lys Thr Glu Gln Leu Ser Cys Met Arg Pro Ala Lys Ser Leu Met
   365 370 375 380
   tcc att tcc aat gct gga tca ggc ttg ctc tcc cac tca tcc acc ctg 1984
   Ser Ile Ser Asn Ala Gly Ser Gly Leu Leu Ser His Ser Ser Thr Leu
   385 390 395
   act ggc tcc ccc atc atg gaa gaa aag cga gac gac aag agc tgg aag 2032
   Thr Gly Ser Pro Ile Met Glu Glu Lys Arg Asp Asp Lys Ser Trp Lys
   400 405 410
   ggg agc cta ggc att ctc ctg ggt gga gac tac cgt gct gaa tat gtc 2080
   Gly Ser Leu Gly Ile Leu Leu Gly Gly Asp Tyr Arg Ala Glu Tyr Val
   415 420 425
   cct tcc aca ccc tcg cct gtg cca ccc tcg act ccc ctg ctc tcg gct 2128
   Pro Ser Thr Pro Ser Pro Val Pro Pro Ser Thr Pro Leu Leu Ser Ala
   430 435 440
   cac tcc aag aca ggc agc cga gac tgc agt acc caa act gaa cgt ggg 2176
   His Ser Lys Thr Gly Ser Arg Asp Cys Ser Thr Gln Thr Glu Arg Gly
   445 450 455 460
   acg gaa tcg aac aaa act gca gct gtt gct ccc atc tct gtt cct gct 2224
   Thr Glu Ser Asn Lys Thr Ala Ala Val Ala Pro Ile Ser Val Pro Ala
   465 470 475
   cca gtt gct gct gcc gcc act gct gcc gcc atc act gcc act gct gcc 2272
   Pro Val Ala Ala Ala Ala Thr Ala Ala Ala Ile Thr Ala Thr Ala Ala
   480 485 490
   acc atc acc acc acc atg gta gct gct gct cca gtt gct gtt gct gct 2320
   Thr Ile Thr Thr Thr Met Val Ala Ala Ala Pro Val Ala Val Ala Ala
   495 500 505
   gct gct gct cca gct gct gct gct gcc ccg tct cca gcc act gcc gct 2368
   Ala Ala Ala Pro Ala Ala Ala Ala Ala Pro Ser Pro Ala Thr Ala Ala
   510 515 520
   gct act gct gct gct gtt tct cca gct gct gct ggt cag att cca gct 2416
   Ala Thr Ala Ala Ala Val Ser Pro Ala Ala Ala Gly Gln Ile Pro Ala
   525 530 535 540
   gct gcc tct gtt gcc tca gct gct gcc gtt gct cct tct gct gct gct 2464
   Ala Ala Ser Val Ala Ser Ala Ala Ala Val Ala Pro Ser Ala Ala Ala
   545 550 555
   gct gct gct gtt cag gtt gct cca gct gct ccg gct cca gtt cca gct 2512
   Ala Ala Ala Val Gln Val Ala Pro Ala Ala Pro Ala Pro Val Pro Ala
   560 565 570
   ccg gct ctg gtt ccg gtt cca gct cca gca gcg gct cag gct tct gct 2560
   Pro Ala Leu Val Pro Val Pro Ala Pro Ala Ala Ala Gln Ala Ser Ala
   575 580 585
   cct gct cag act cag gca cca act tca gct ccg gct gtg gct cca act 2608
   Pro Ala Gln Thr Gln Ala Pro Thr Ser Ala Pro Ala Val Ala Pro Thr
   590 595 600
   cca gct cca act cca act cca gct gtg gct cag gct gag gtt cct gca    2656
   Pro Ala Pro Thr Pro Thr Pro Ala Val Ala Gln Ala Glu Val Pro Ala
   605                 610                 615                 620
   agt cca gct acc ggt cct gga cca cat cgt ttg tct ata cca agt ttg    2704
   Ser Pro Ala Thr Gly Pro Gly Pro His Arg Leu Ser Ile Pro Ser Leu
                   625                 630                 635
   acc tgc aat cca gac aaa aca gat ggg cct gtg ttc cac tcc aat act    2752
   Thr Cys Asn Pro Asp Lys Thr Asp Gly Pro Val Phe His Ser Asn Thr
               640                 645                 650
   ctg gaa aga aaa act ccc att cag atc ctg gga caa gag cct gat gca    2800
   Leu Glu Arg Lys Thr Pro Ile Gln Ile Leu Gly Gln Glu Pro Asp Ala
           655                 660                 665
   gag atg gtg gaa tat ctc atc taa acggccaaat caagagctgc agattatcag   2854
   Glu Met Val Glu Tyr Leu Ile
       670                 675
   caaaaatgct tttaatcatt ttcccccttt tattggttct tgttttgagg gtgaggacaa 2914
   gggttgtggg gaggggatgt tttttaacag gactttttat tggaacaatg tactacttga 2974
   gtaataccat gtgaacacca gtctattttg gtatgcttag ggagtacctc taaagacaga 3034
   ttaatcagaa tgtgctctaa agcttattgt ttgaatttat acgaatactg ggactgttaa 3094
   caggtggcta tacatcgacg ttttcaatgt gcttaaattt gtttaaattt tccatattct 3154
   agatcatttt ttattgaaga gcacagtatg tgtggaagac agtgtataac acgtagtttg 3214
   gaagtgggaa gctagagaga attgagtgtg tgctgttttg tatagttact atcctgtgca 3274
   gcagctggag aaagcactca cctcaggctt acaaaaggga atagtttcag gagctatgta 3334
   agctggaaaa aaggtaggga gttttggggt gcagaagggt actggagcta atttttcttt 3394
   ccagtttccc agctaccctg ccccagggaa ttgtgtttgt cttcatttca gtggtgcttt 3454
   ggaaatggat tcttttggtt ccctcctgga ggttcataca ttcatatata tgctctggag 3514
   taatttatgc atttggataa ttaatatatt gctttcagat gctgggagag tacattaact 3574
   gagtgatgcg caacttcctc tctcttaggg aattagacca tcagaggcct tgatggagag 3634
   ttgcatgggg tgctatatgc agacttccat ggtttgtgtg tagccatgaa cacagcttgc 3694
   ttgcatttag taagaccaat cagcttagtg tttatttctt ctacagcaca gattcactgg 3754
   ctgggtctcc agtctcaaat tgccaatcat ttgcaaagtg aggaaggatc tttgttgaca 3814
   ggttgaatgc tttgaatttc tggtgactac tttgaaataa cttgttttgt ttgtcaaatt 3874
   ctaagcatat gtcttaaaag gcatttttga ctatcacctc caagggaata gcttgagaaa 3934
   cccaaagtac tatgctgcag tcgggggaga ggtggattgc agcagtatcc tcaactacct 3994
   cttctcactg tcagtgacac catcttggaa tacctttggg aagcagcagg aaatgtgcat 4054
   gtgggtagag atcaaaggag gcaatggctc caagccttgc catagggctg cctccaagga 4114
   cacagaagga tgccagttgc cacaggtccc tgccctgtgt cacctgtctg cccttcatta 4174
   aggtgagaaa tctgcagata gcatcattaa gatcagtttt aaggggtata gggagggtga 4234
   gggaagtggg gggtgttagg taagggttgg gggtagaggt tttgggatgt cttagttaga 4294
   aaccagatta atagaagagt aggcctgata tattacatca tgagccatag tggtgggaaa 4354
   gaactttagc aatatagccc tacctcctca ttttagtgat gaggaatctg agaactggag 4414
   aggttcagtg actttttgaa agtcatacaa cacagctaac cattatgcca atcaccatgc 4474
   ttattttggg aaactcttta tcttttttaa attccatttt atgaaaaggc atcttcatgg 4534
   tccagggaat atgtatcttg taaaatgtac ctggttggag tagcttgtcc agtcttgaca 4594
   aactactgaa tttctgtctt gcctctcctt cagtgccttt taaaaggttt tcccttttct 4654
   gatctgcatt tcaacataga gtcacataaa tgtccccctg agaaaccaat cccacttctt 4714
   tctaggagat tgggtatctt agataatctt ttggggttcc tctgtgagta taggaatggt 4774
   atccttccta attatcttcc aaaggaatta ttttgtgtgt gtgcctgtgt gtgtgtagag 4834
   acataaagga gggtgatgtg attttcagct agtcctttca cattttcaat aatgaggtaa 4894
   tcatgttaca tacacattag tcctcagtta taaagtgaat ctcagataga aattaaaagt 4954
   gcagttgtgt taagactctt tcatactacc ctttagtcat aaggagaaaa aaacactcaa 5014
   atagtagaag cagcaagtag caaacttcag gagagctact ttctatccaa ataatttaaa 5074
   aaacactttt cacctactcc tttcatggtt ataacacatt ggcagacttt ttgctggctc 5134
   tgggagccat gattttaatc acattctgca aggtgacaaa tgtcatacat tccacattgt 5194
   gtggtagcca tctctttaga ctcatgtgtt ttggggaaag gaagaagttc ttggctgagt 5254
   actattttga actttccaga accctctcac accagagaca gttcttctct gttcagtttc 5314
   caatccccga taatttgcta aaataacatt gtacatccaa gagagggaag aagagtatgt 5374
   cagtatatta tgcagaagat agatacagcc ttttcagaag atctccacta gtttttgttc 5434
   caaaaattca agtttatggg agaaatctca attagccacc ttttcacagt tgtgtggata 5494
   taacatttgg gggatctttc tggactccta cctatctgtg cattttaccg gcacctcagg 5554
   aaaggagggt gaccaggttg tcttagcttg tactgcttgg tgatctctga ggaccttcta 5614
   attcagttgt accccagtgt tccatgtata gaaaaacttc attagaacaa actttacttg 5674
   atatgaaact cctattaaca gtcttttttt gaaataaaaa gtagcttgag ctttctttta 5734
   aaatcatgta tcttgattgt tgatttaatg aaggatttcc ttttaatgct gcttttgagc 5794
   ttcaaggtaa taggacagca ggaacctaaa atatctgcca tcatctgcca taggaaagat 5854
   acccagagac ccatcatgtt ctctttttgt tgttacactg ttgggtgggt ataacaattg 5914
   gaaaatgaac aaactgattg attgtgcaaa ctacttttta tgacaagcct aaaccctcat 5974
   aatgcggcag cttaaagtgt atacatatgc actaactttg atcaattata ttctcatatc 6034
   tgttagctac acagtctcct attatctcaa ttgcttatgt gcatatggaa tatgttactt 6094
   aaaacgtgtg cattcttact gaaaatgttt tcaaaggaag gtatcagctg tgggctaatt 6154
   gccaccaatt tcagcctgcc acgattcttg gaaatatgtc ttccaagtgc catccatcat 6214
   cagtaggaca agtgtcggga gtttgtttat ttttttccag tagcaacgat gggttacatg 6274
   gagccatgaa acctccttct ggcctcccct gtgattaatg gcatgtgttt gtaaaatgga 6334
   tagctggggt tggcagatgg ctagagaaga atcgcctttg gtttaaaatg tatgtggtcc 6394
   cctaatgatt gtgaccccat tctgtaatca actgagctag ttccaataaa gttaagcagg 6454
   tttaaatcc 6463
<210> 2
   <211> 675
   <212> PRT
   <213> Homo sapiens
<400> 2
   Met Pro Arg Ala Gln Pro Ser Ser Ala Ser Tyr Gln Pro Val Pro Ala
   1 5 10 15
   Asp Pro Phe Ala Ile Val Ser Arg Ala Gln Gln Met Val Glu Ile Leu
   20 25 30
   Ser Asp Glu Asn Arg Asn Leu Arg Gln Glu Leu Glu Gly Cys Tyr Glu
   35 40 45
   Lys Val Ala Arg Leu Gln Lys Val Glu Thr Glu Ile Gln Arg Val Ser
   50 55 60
   Glu Ala Tyr Glu Asn Leu Val Lys Ser Ser Ser Lys Arg Glu Ala Leu
   65 70 75 80
   Glu Lys Ala Met Arg Asn Lys Leu Glu Gly Glu Ile Arg Arg Met His
   85 90 95
   Asp Phe Asn Arg Asp Leu Arg Glu Arg Leu Glu Thr Ala Asn Lys Gln
   100 105 110
   Leu Ala Glu Lys Glu Tyr Glu Gly Ser Glu Asp Thr Arg Lys Thr Ile
   115 120 125
   Ser Gln Leu Phe Ala Lys Asn Lys Glu Ser Gln Arg Glu Lys Glu Lys
   130 135 140
   Leu Glu Ala Glu Leu Ala Thr Ala Arg Ser Thr Asn Glu Asp Gln Arg
   145 150 155 160
   Arg His Ile Glu Ile Arg Asp Gln Ala Leu Ser Asn Ala Gln Ala Lys
   165 170 175
   Val Val Lys Leu Glu Glu Glu Leu Lys Lys Lys Gln Val Tyr Val Asp
   180 185 190
   Lys Val Glu Lys Met Gln Gln Ala Leu Val Gln Leu Gln Ala Ala Cys
   195 200 205
   Glu Lys Arg Glu Gln Leu Glu His Arg Leu Arg Thr Arg Leu Glu Arg
   210 215 220
   Glu Leu Glu Ser Leu Arg Ile Gln Gln Arg Gln Gly Asn Cys Gln Pro
   225 230 235 240
   Thr Asn Val Ser Glu Tyr Asn Ala Ala Ala Leu Met Glu Leu Leu Arg
   245 250 255
   Glu Lys Glu Glu Arg Ile Leu Ala Leu Glu Ala Asp Met Thr Lys Trp
   260 265 270
   Glu Gln Lys Tyr Leu Glu Glu Asn Val Met Arg His Phe Ala Leu Asp
   275 280 285
   Ala Ala Ala Thr Val Ala Ala Gln Arg Asp Thr Thr Val Ile Ser His
   290 295 300
   Ser Pro Asn Thr Ser Tyr Asp Thr Ala Leu Glu Ala Arg Ile Gln Lys
   305 310 315 320
   Glu Glu Glu Glu Ile Leu Met Ala Asn Lys Arg Cys Leu Asp Met Glu
   325 330 335
   Gly Arg Ile Lys Thr Leu His Ala Gln Ile Ile Glu Lys Asp Ala Met
   340 345 350
   Ile Lys Val Leu Gln Gln Arg Ser Arg Lys Glu Pro Ser Lys Thr Glu
   355 360 365
   Gln Leu Ser Cys Met Arg Pro Ala Lys Ser Leu Met Ser Ile Ser Asn
   370 375 380
   Ala Gly Ser Gly Leu Leu Ser His Ser Ser Thr Leu Thr Gly Ser Pro
   385 390 395 400
   Ile Met Glu Glu Lys Arg Asp Asp Lys Ser Trp Lys Gly Ser Leu Gly
   405 410 415
   Ile Leu Leu Gly Gly Asp Tyr Arg Ala Glu Tyr Val Pro Ser Thr Pro
   420 425 430
   Ser Pro Val Pro Pro Ser Thr Pro Leu Leu Ser Ala His Ser Lys Thr
   435 440 445
   Gly Ser Arg Asp Cys Ser Thr Gln Thr Glu Arg Gly Thr Glu Ser Asn
   450 455 460
   Lys Thr Ala Ala Val Ala Pro Ile Ser Val Pro Ala Pro Val Ala Ala
   465 470 475 480
   Ala Ala Thr Ala Ala Ala Ile Thr Ala Thr Ala Ala Thr Ile Thr Thr
   485 490 495
   Thr Met Val Ala Ala Ala Pro Val Ala Val Ala Ala Ala Ala Ala Pro
   500 505 510
   Ala Ala Ala Ala Ala Pro Ser Pro Ala Thr Ala Ala Ala Thr Ala Ala
   515 520 525
   Ala Val Ser Pro Ala Ala Ala Gly Gln Ile Pro Ala Ala Ala Ser Val
   530 535 540
   Ala Ser Ala Ala Ala Val Ala Pro Ser Ala Ala Ala Ala Ala Ala Val
   545 550 555 560
   Gln Val Ala Pro Ala Ala Pro Ala Pro Val Pro Ala Pro Ala Leu Val
   565 570 575
   Pro Val Pro Ala Pro Ala Ala Ala Gln Ala Ser Ala Pro Ala Gln Thr
   580 585 590
   Gln Ala Pro Thr Ser Ala Pro Ala Val Ala Pro Thr Pro Ala Pro Thr
   595 600 605
   Pro Thr Pro Ala Val Ala Gln Ala Glu Val Pro Ala Ser Pro Ala Thr
   610 615 620
   Gly Pro Gly Pro His Arg Leu Ser Ile Pro Ser Leu Thr Cys Asn Pro
   625 630 635 640
   Asp Lys Thr Asp Gly Pro Val Phe His Ser Asn Thr Leu Glu Arg Lys
   645 650 655
   Thr Pro Ile Gln Ile Leu Gly Gln Glu Pro Asp Ala Glu Met Val Glu
   660 665 670
   Tyr Leu Ile
   675
<210> 3
   <211> 675
   <212> PRT
   <213> Homo sapiens
<220>
   <221> VARIANT
   <222> ()..)
   <223> Residue 135 = Asn, Ser or Asp
<220>
   <221> VARIANT
   <222> () .. (150)
   <223> Residues 148-150 = Glu-Leu-Ala or Thr-Thr-Pro
<400> 3
   Met Pro Arg Ala Gln Pro Ser Ser Ala Ser Tyr Gln Pro Val Pro Ala
   1 5 10 15
   Asp Pro Phe Ala Ile Val Ser Arg Ala Gln Gln Met Val Glu Ile Leu
   20 25 30
   Ser Asp Glu Asn Arg Asn Leu Arg Gln Glu Leu Glu Gly Cys Tyr Glu
   35 40 45
   Lys Val Ala Arg Leu Gln Lys Val Glu Thr Glu Ile Gln Arg Val Ser
   50 55 60
   Glu Ala Tyr Glu Asn Leu Val Lys Ser Ser Ser Lys Arg Glu Ala Leu
   65 70 75 80
   Glu Lys Ala Met Arg Asn Lys Leu Glu Gly Glu Ile Arg Arg Met His
   85 90 95
   Asp Phe Asn Arg Asp Leu Arg Glu Arg Leu Glu Thr Ala Asn Lys Gln
   100 105 110
   Leu Ala Glu Lys Glu Tyr Glu Gly Ser Glu Asp Thr Arg Lys Thr Ile
   115 120 125
   Ser Gln Leu Phe Ala Lys Xaa Lys Glu Ser Gln Arg Glu Lys Glu Lys
   130 135 140
   Leu Glu Ala Xaa Xaa Xaa Thr Ala Arg Ser Thr Asn Glu Asp Gln Arg
   145 150 155 160
   Arg His Ile Glu Ile Arg Asp Gln Ala Leu Ser Asn Ala Gln Ala Lys
   165 170 175
   Val Val Lys Leu Glu Glu Glu Leu Lys Lys Lys Gln Val Tyr Val Asp
   180 185 190
   Lys Val Glu Lys Met Gln Gln Ala Leu Val Gln Leu Gln Ala Ala Cys
   195 200 205
   Glu Lys Arg Glu Gln Leu Glu His Arg Leu Arg Thr Arg Leu Glu Arg
   210 215 220
   Glu Leu Glu Ser Leu Arg Ile Gln Gln Arg Gln Gly Asn Cys Gln Pro
   225 230 235 240
   Thr Asn Val Ser Glu Tyr Asn Ala Ala Ala Leu Met Glu Leu Leu Arg
   245 250 255
   Glu Lys Glu Glu Arg Ile Leu Ala Leu Glu Ala Asp Met Thr Lys Trp
   260 265 270
   Glu Gln Lys Tyr Leu Glu Glu Asn Val Met Arg His Phe Ala Leu Asp
   275 280 285
   Ala Ala Ala Thr Val Ala Ala Gln Arg Asp Thr Thr Val Ile Ser His
   290 295 300
   Ser Pro Asn Thr Ser Tyr Asp Thr Ala Leu Glu Ala Arg Ile Gln Lys
   305 310 315 320
   Glu Glu Glu Glu Ile Leu Met Ala Asn Lys Arg Cys Leu Asp Met Glu
   325 330 335
   Gly Arg Ile Lys Thr Leu His Ala Gln Ile Ile Glu Lys Asp Ala Met
   340 345 350
   Ile Lys Val Leu Gln Gln Arg Ser Arg Lys Glu Pro Ser Lys Thr Glu
   355 360 365
   Gln Leu Ser Cys Met Arg Pro Ala Lys Ser Leu Met Ser Ile Ser Asn
   370 375 380
   Ala Gly Ser Gly Leu Leu Ser His Ser Ser Thr Leu Thr Gly Ser Pro
   385 390 395 400
   Ile Met Glu Glu Lys Arg Asp Asp Lys Ser Trp Lys Gly Ser Leu Gly
   405 410 415
   Ile Leu Leu Gly Gly Asp Tyr Arg Ala Glu Tyr Val Pro Ser Thr Pro
   420 425 430
   Ser Pro Val Pro Pro Ser Thr Pro Leu Leu Ser Ala His Ser Lys Thr
   435 440 445
   Gly Ser Arg Asp Cys Ser Thr Gln Thr Glu Arg Gly Thr Glu Ser Asn
   450 455 460
   Lys Thr Ala Ala Val Ala Pro Ile Ser Val Pro Ala Pro Val Ala Ala
   465 470 475 480
   Ala Ala Thr Ala Ala Ala Ile Thr Ala Thr Ala Ala Thr Ile Thr Thr
   485 490 495
   Thr Met Val Ala Ala Ala Pro Val Ala Val Ala Ala Ala Ala Ala Pro
   500 505 510
   Ala Ala Ala Ala Ala Pro Ser Pro Ala Thr Ala Ala Ala Thr Ala Ala
   515 520 525
   Ala Val Ser Pro Ala Ala Ala Gly Gln Ile Pro Ala Ala Ala Ser Val
   530 535 540
   Ala Ser Ala Ala Ala Val Ala Pro Ser Ala Ala Ala Ala Ala Ala Val
   545 550 555 560
   Gln Val Ala Pro Ala Ala Pro Ala Pro Val Pro Ala Pro Ala Leu Val
   565 570 575
   Pro Val Pro Ala Pro Ala Ala Ala Gln Ala Ser Ala Pro Ala Gln Thr
   580 585 590
   Gln Ala Pro Thr Ser Ala Pro Ala Val Ala Pro Thr Pro Ala Pro Thr
   595 600 605
   Pro Thr Pro Ala Val Ala Gln Ala Glu Val Pro Ala Ser Pro Ala Thr
   610 615 620
   Gly Pro Gly Pro His Arg Leu Ser Ile Pro Ser Leu Thr Cys Asn Pro
   625 630 635 640
   Asp Lys Thr Asp Gly Pro Val Phe His Ser Asn Thr Leu Glu Arg Lys
   645 650 655
   Thr Pro Ile Gln Ile Leu Gly Gln Glu Pro Asp Ala Glu Met Val Glu
   660 665 670
   Tyr Leu Ile
   675
<210> 4
   <211> 143
   <212> PRT
   <213> Homo sapiens
<400> 4
   Glu Ser Asn Lys Thr Ala Ala Val Ala Pro Ile Ser Val Pro Ala Pro
   1 5 10 15
   Val Ala Ala Ala Ala Thr Ala Ala Ala Ile Thr Ala Thr Ala Ala Thr
   20 25 30
   Ile Thr Thr Thr Met Val Ala Ala Ala Pro Val Ala Val Ala Ala Ala
   35 40 45
   Ala Ala Pro Ala Ala Ala Ala Ala Pro Ser Pro Ala Thr Ala Ala Ala
   50 55 60
   Thr Ala Ala Ala Val Ser Pro Ala Ala Ala Gly Gln Ile Pro Ala Ala
   65 70 75 80
   Ala Ser Val Ala Ser Ala Ala Ala Val Ala Pro Ser Ala Ala Ala Ala
   85 90 95
   Ala Ala Val Gln Val Ala Pro Ala Ala Pro Ala Pro Val Pro Ala Pro
   100 105 110
   Ala Leu Val Pro Val Pro Ala Pro Ala Ala Ala Gln Ala Ser Ala Pro
   115 120 125
   Ala Gln Thr Gln Ala Pro Thr Ser Ala Pro Ala Val Ala Pro Thr
   130 135 140
<210> 5
   <211> 31
   <212> DNA
   <213> Artificial Sequence
<220>
   <223> Description of Artificial Sequence:oligonucleotide primer for PCR reaction
<400> 5
   tacggatccg aatcgaacaa aactgcagct g 31
<210> 6
   <211> 32
   <212> DNA
   <213> Artificial Sequence
<220>
   <223> Description of Artificial Sequence:oligonucleotide primer for PCR reaction
<400> 6
   atactcgagt catggagctg gagttggagc ca 32
<210> 7
   <211> 22
   <212> DNA
   <213> Artificial Sequence
<220>
   <223> Description of Artificial Sequence:oligonucleotide primer for PCR reaction
<400> 7
   gtttgacctg caatccagac aa 22
<210> 8
   <211> 21
   <212> DNA
   <213> Artificial Sequence
<220>
   <223> Description of Artificial Sequence:oligonucleotide primer for PCR reaction
<400> 8
   cccaggatct gaatgggagt t 21
<210> 9
   <211> 25
   <212> DNA
   <213> Artificial Sequence
<220>
   <223> Description of Artificial Sequence:oligonucleotide primer for PCR reaction
<400> 9
   cagatgggcc tgtgttccac tccaa 25
<210> 10
   <211> 24
   <212> DNA
   <213> Artificial Sequence
<220>
   <223> Description of Artificial Sequence:oligonucleotide primer for RACE PCR reaction
<400> 10
   gctgacagtt gccctgacgc tgct 24
<210> 11
   <211> 24
   <212> DNA
   <213> Artificial Sequence
<220>
   <223> Description of Artificial Sequence:oligonucleotide primer for RACE PCR reaction
<400> 11
   cggagacggt gctctagctg ctca 24
<210> 12
   <211> 27
   <212> DNA
   <213> Artificial Sequence
<220>
   <223> Description of Artificial Sequence:oligonucleotide primer for RACE PCR reaction
<400> 12
   tccttccaac tcttgcctca agttccg 27
<210> 13
   <211> 24
   <212> DNA
   <213> Artificial Sequence
<220>
   <223> Description of Artificial Sequence:oligonucleotide primer for RACE PCR reaction
<400> 13
   ggtggcagcg gacaggcagg atac 24
<210> 14
   <211> 24
   <212> DNA
   <213> Artificial Sequence
<220>
   <223> Description of Artificial Sequence:oligonucleotide primer for RACE PCR reaction
<400> 14
   gaggcggaga gaactaagag aaga                                                    24
<210> 15
   <211> 24
   <212> DNA
   <213> Artificial Sequence
<220>
   <223> Description of Artificial Sequence:oligonucleotide primer for RACE PCR reaction
<400> 15
   gagcggagat ggaggagtaa ttca                                                    24